# EUROPEAN PATENT APPLICATION

(11) **EP 2 423 842 A1**
(43) Date of publication of application: **29.02.2012**
(21) Application number: 11004212.4
(22) Date of filing: 20.05.2011
(51) Int. Cl.: G06F 19/14

(54) **Information processing apparatus, method and program for phylogeny analysis**

(30) Priority: 25.05.2010 JP 2010119915; 09.02.2011 JP 2011026371
(62) Divisional of application: 11007577.7
(71) Applicant: Sony Corporation, Tokyo 108-0075 (JP)
(72) Inventor: Yoshida, Kaoru, Tokyo (JP)
(74) Representative: Müller - Hoffmann & Partner

(57) **Abstract**

There is provided an information processing including a data acquiring unit which acquires string data representing a string of one or more characters, and a phylogeny analyzing unit which analyzes the string data acquired by the data acquiring unit to extract homologous string pieces in a string represented by the string data and performs phylogeny analysis based on the regional relationship and homological relationship of the extracted homologous string pieces.

## Description

### BACKGROUND

Embodiments of the present invention relate to an information processing apparatus, an information processing method, and a program.

### Description of the Related Art

In the genetic engineering with the insertion of a foreign gene, in order to preserve a host function, the inhibition of control of an operon (a plurality of continuous gene clusters which are transcriptionally controlled by a pair of promotor and terminator) placed around the insertion position of the foreign gene should be avoided. Thus, it is necessary to identify a promotor region, an enhancer region, and a terminator region. Software and servers for extracting and analyzing these elements are available. Those regions can be identified based on data about a local sequence around a candidate insertion position.

In addition to identification of the above regions, it is necessary to previously examine an unstable region on a genome harboring the genetic information in a cell, i.e., a region where genome rearrangement is likely generated. This is because the insertion of the foreign gene into the unstable region may cause chromosome rearrangement such as deletion, modification, and replication.

As a molecular mechanism associated with the genomic rearrangement, there is a phenomenon referred to as "homologous recombination" which is generated when two homologous DNA fragments are aligned. When the homologous DNA fragments are present in the same direction, a DNA molecule is cleaved or two DNA molecules are fused. When the homologous DNA fragments are present in the opposite direction, a region between the homologous DNA fragments is transposed. When the possibility of such homologous recombination is concerned, it is desirable that a foreign gene and a gene homologous to the foreign gene are not present close to each other.

The cleavage and integration of DNA by homologous recombination leads to the birth of molecules which are transpositioned, moved, and parasitized on chromosomes, into or out of the chromosomes, or between cells, like transposons, plasmids, and phages. In addition to the mutation in each gene, it is considered that the gain, loss, and modification of nucleic acid sequences through these mobile molecules play a large role in the adaptation to environment, diversification, and evolution of organisms.

The unstable region on the present chromosome and the transition of the past chromosome rearrangement are revealed by investigating whether these transpositioned, moved, and parasitized molecules are currently present in cells, or whether the remaining of the molecules as a trace of the presence in the past is present, or whether phylogenetic properties are observed in the remaining of the molecules. An investigation of phylogenetic properties and structuralization of mobile molecules for different strains or types leads to the elucidation of biological evolution. It is possible to reveal the relationship of biological evolution by analyzing such data, so that a diagram referred to as a phylogenetic tree can be created. For example, Japanese Patent Application Laid-Open No. 5-128171 suggests a device for generating phylogenetic tree information using a similarity matrix formed of the similarity between the arrangements preliminarily obtained.

1.6 million kinds of organisms have been identified since the life began on the earth about 3.8 billion years ago. Although it is estimated that 1 to 10 million kinds of bacteria exist including eubacteria and archaebacteria, the identified bacteria are only about 10 thousand kinds. The kind of organisms was defined in accordance with the morphology, habitat and mating system at one time. Five kingdoms of: Animalia, Plantae, Fungi, Protozoa, and Monera have been used for a long time. However, three domains of: Archaea, Bacteria, and Eukarya have been recently put forward based on molecular phylogenic analysis using gene sequences preserved in all organisms such as 16S rRNA.

Eubacteria have small chromosomes with less than 10 Mbp and their 16S rRNA sequences are highly homologous to each other. In spite of the preservation of many common genes, their arrangements on chromosomes are very different from one another. Despite the fact that a difference in gene information between chromosomes of humans and chimpanzees diverged from a common ancestor about 5 million years ago is just 1.23% (about 37 million bases), there are regions with significant difference in the structure and arrangement. The chromosome rearrangement caused by an abnormal repetition of the repeated sequence or modification, including microsatellites distributed over about 1.2 million places on human chromosomes, is considered as the cause of pathology, which is used for diagnosis for hereditary or acquired cancer, psychiatric disorder, immune abnormality, metabolic anomaly or the like.

### SUMMARY OF THE INVENTION

As described above, the research of biological evolution has been recently promoted using a phylogenetic analysis technique based on the modification on a gene sequence such as 16S rRNA, and a phylogeny analysis software developed for the technique is available. Simple repetitive elements and continuous repetitive elements adjacent to each other were searched to compile a database.

However, a procedure and analysis tools for grasping the structure of the whole genome from the viewpoint of distribution and phylogeny of homologous domains and estimating the transitions of chromosome rearrangement and the unstable regions have not been developed so far.

In light of the foregoing, it is desirable to provide an information processing apparatus, an information processing method, and a program, capable of estimating the transition of chromosome rearrangement and the unstable region by analyzing the structure of a genome for general purpose, not based on the modification on a specific gene sequence,.

In order to solve the above issue, according to an embodiment of the present invention, there is provided an information processing apparatus including a data acquiring unit which acquires string data representing a string of one or more characters; and a phylogeny analyzing unit which analyzes the string data acquired by the data acquiring unit to extract homologous string pieces in a string represented by the string data and performs phylogeny analysis based on the present location and homological relationship of the extracted homologous string pieces.

The phylogeny analyzing unit may further preferably include a sequence aligning unit which performs sequence alignment on a plurality of string data and calculates the similarity measure between the string data, a homologous domain extracting unit which extracts homologous domains including homologous section information representing sections of the homologous string pieces and homological relationship information represented by using at least one of the direction and the degree of similarity of the sections of the homologous string pieces using the sequence alignment result by the sequence aligning unit, a homological group analyzing unit which analyzes the homologous domains dispersedly present in the same string data or between the different string data and groups homologous domains having the common homologous section information among the homologous domains into a homological group, a regional group analyzing unit which analyzes the homologous domains dispersedly present in the same string data or between the different string data and produces a regional group representing inclusion and overlap relationships between the homologous domains, and a family analyzing unit which analyzes a family of the homologous string pieces based on information about the homological group grouped by the homological group analyzing unit and information about the regional group produced by the regional group analyzing unit.

The regional group analyzing unit may set a region obtained by adding a predetermined length of margin section to both ends of the sections of the homologous string pieces as a small region, set the small region which is included in neither of the other small regions as a middle region, and set a set of the middle region overlapped as a large region.

The family analyzing unit may analyze the small region, the middle region, and the large region produced by the regional group analyzing unit and estimate a small region family representing a family of the small region, a middle region family representing a family of the middle region, and a large region family representing a family of the large region.

The phylogeny analyzing unit may further include a family subdividing unit which subdivides the large region family.

The phylogeny analyzing unit may further include a direction determining unit which determines the alignment direction of a section of the homologous string piece which belong to the same homological group and the same family among the sections of the homologous string pieces belonging to the small region, the middle region, and the large region.

The information processing apparatus may further include a function analyzing unit which analyzes a function of the homologous domain based on the analysis result by the phylogeny analyzing unit.

The phylogeny analyzing unit may further perform the phylogeny analysis on the string data representing the phylogeny analysis result of the extracted homologous string pieces.

In order to solve the above issue, according to another embodiment of the present invention, there is provided an information processing method including the steps of: acquiring string data representing a string of one or more characters; analyzing the acquired string data to extract homologous string pieces in a string represented by the string data; and performing phylogeny analysis based on the present location and homological relationship of the extracted homologous string pieces.

In order to solve the above issue, according to another embodiment of the present invention, there is provided a program for causing a computer to achieve: a data acquisition function for acquiring string data representing a string of one or more characters; and a phylogeny analysis function for analyzing the string data acquired by the data acquisition function, extracting homologous string pieces in a string represented by the string data, and performing phylogeny analysis based on the regional relationship and homological relationship of the extracted homologous string pieces.

According to the embodiments of the present invention described above, the transitions of chromosome rearrangement and the unstable regions can be estimated by analyzing the genome structure from the viewpoint of distribution and phylogeny of homologous domains.

### BRIEF DESCRIPTION OF THE DRAWINGS

FIG. 1 is an explanatory diagram for explaining a homologous recombination;
FIG. 2 is a block diagram showing the configuration of an information processing apparatus according to a first embodiment of the present invention;
FIG. 3 is an explanatory diagram for explaining homologous domain function analysis processing according to the first embodiment;
FIG. 4 is a block diagram showing the configuration of a homologous domain phylogeny analyzing unit according to the first embodiment;
FIG. 5A is an explanatory diagram for explaining homologous domain phylogeny analysis processing according to the first embodiment;
FIG. 5B is an explanatory diagram for explaining homologous domain phylogeny analysis processing according to the first embodiment;
FIG. 5C is an explanatory diagram for explaining homologous domain phylogeny analysis processing according to the first embodiment;
FIG. 5D is an explanatory diagram for explaining homologous domain phylogeny analysis processing according to the first embodiment;
FIG. 5E is an explanatory diagram for explaining homologous domain phylogeny analysis processing according to the first embodiment;
FIG. 6 is a flow chart showing the flow of the homologous domain phylogeny analysis processing according to the first embodiment;
FIG. 7 is a flow chart showing the flow of the homologous domain function analysis processing according to the first embodiment;
FIG. 8 is an explanatory diagram showing an example of software for analyzing genome structure using the homologous domain phylogeny analysis processing and the homologous domain function analysis processing according to the first embodiment;
FIG. 9 is a block diagram showing the hardware configuration of an information processing apparatus according to the embodiments of the present invention;
FIG. 10A is an explanatory view showing an applied example of the information processing apparatus according to the first embodiment of the present invention;
FIG. 10B is an explanatory diagram showing an applied example of the information processing apparatus according to the first embodiment;
FIG. 11A is an explanatory diagram showing an applied example of the information processing apparatus according to the first embodiment;
FIG. 11B is an explanatory diagram showing an applied example of the information processing apparatus according to the first embodiment;
FIG. 11C is an explanatory diagram showing an applied example of the information processing apparatus according to the first embodiment;
FIG. 11D is an explanatory diagram showing an applied example of the information processing apparatus according to the first embodiment;
FIG. 12A is an explanatory diagram showing an applied example of the information processing apparatus according to the first embodiment;
FIG. 12B is an explanatory diagram showing an applied example of the information processing apparatus according to the first embodiment;
FIG. 13 is an explanatory diagram showing an applied example of the information processing apparatus according to the first embodiment; and
FIG. 14 is an explanatory diagram showing an applied example of the information processing apparatus according to the first embodiment.

### DETAILED DESCRIPTION OF THE EMBODIMENT(S)

Hereinafter, preferred embodiments of the present invention will be described in detail with reference to the appended drawings. Note that, in this specification and the appended drawings, structural elements that have substantially the same function and structure are denoted with the same reference numerals, and repeated explanation of these structural elements is omitted.

In addition, the explanation will be given in the following order.
(1) Hhomologous recombination
(2) First embodiment
(2-1) Configuration of information processing apparatus
(2-2) Flow of method of analyzing homologous domain phylogeny
(2-3) Flow of method of analyzing homologous domain function
(2-4) One example of software for analyzing genome structure
(2-5) First modified example
(3) Hardware configuration of information processing apparatus according to the embodiment of the present invention
(4) Applied example
(5) Summary

### (Homologous recombination)

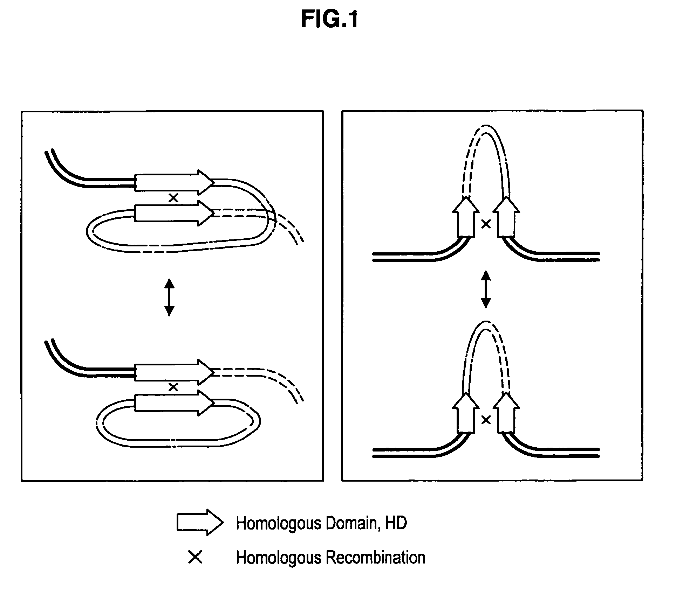

Before explaining the information processing apparatus and the method of analyzing phylogeny according to the embodiments of the present invention, the phenomenon of homologous recombination will be briefly explained with reference to FIG. 1. FIG. 1 is an explanatory view for explaining the homologous recombination.

As shown in FIG. 1, as a molecular mechanism associated with the genomic rearrangement, there is a phenomenon referred to as "homologous recombination" which is generated when two homologous DNA fragments are aligned. Here, the "homologous DNA fragments" mean that their DNA sequences are similar because they have a common evolutionary origin. Since such homologous DNA fragments have similar base sequences, they have similar functions each other in many cases.

When the homologous DNA fragments are present in the same direction, a DNA molecule is divided or two DNA molecules are fused. This case corresponds to the case shown in FIG. 1A. In FIG. 1A, the recombination of DNA fragments occurs in the region where the homologous DNA fragments are present. As a result, a DNA molecule is divided into two DNA molecules. Like the example shown in FIG. 1B, when the homologous DNA fragments are present in the opposite direction, the region between the homologous DNA fragments is reversed.

Thus, a sequence in a portion where recombination is generated by such homologous recombination is different from the sequence before the recombination. The DNA sequence corresponding to the region where recombination is generated may have a different function from that before the recombination or may lose a function one has had before the recombination.

Conversely, in a region where the homologous DNA fragments are present near each other, there is a higher possibility that the DNA sequence changes by the homologous recombination described above. Therefore, it is preferable that when a foreign gene is inserted into a certain gene, the foreign gene is inserted into a region where a gene homologous to the foreign gene is not present near the foreign gene to prevent any change in the foreign gene from occurring by homologous recombination.

Here, it is briefly discussed what kind of range the term "near" in the gene sequence includes. A circular DNA molecule is in a supercoil state where the DNA molecule is multiply twisted to minimize free energy. Accordingly, even when a gene homologous to the circular DNA molecule is positioned at the farthest place from the circular DNA molecule on the sequence (for example, opposite side of the ring), the gene may be near the circular DNA molecule in the supercoil state. When different circular DNA molecules move into the cell, they may be adjacent to each other. Therefore, it may be assumed that the above-described homologous recombination may occur on all of the intracellular nucleic acid molecules.

As shown in FIG. 1, the cleavage and integration of DNA by homologous recombination leads to the birth of molecules which are transpositioned, moved, and parasitized on chromosomes, into or out of the chromosomes, or between cells, like transposons, plasmids, and phages.

In 1940, Barbara McClintock first discovered a transposon being transpositioned on chromosomes and referred to as a maize pigment controlling element. A DNA-type transposon has an inverse repetitive element at the end and codes for an enzyme called as transposase between the ends of the transposon. This enzyme plays a role in the process of recognizing repetitive elements at both ends, recombining these elements, separating a transposon from the present position on a chromosome, and inserting it into another position.

Therefore, the transposon insertion inhibits the expression of a gene at the insertion position and a related gene.

In 1946, Joshua Lederberg discovered a plasmid moving into or out of chromosomes and moving between bacteria in Escherichia coli. Escherichia coli has F and R factor plasmids in a cell, in addition to a main chromosome. These factors do not have genes necessary for cell growth. However, these factors have a gene necessary for the formation and conjugation of single stranded DNA, such as an ssb (single-stranded DNA binding protein) gene. Thus, the factors move into other cells through sex pili as a single stranded DNA and they are replicated into double stranded DNA fragments through the rolling cycle mechanism in the cells to which the factors have been transferred. There are two types of the F factors: an F+ factor typically present separately from a chromosome and an Hfr factor inserting into chromosome. The R factor has a drug resistance gene.

A phage moving into or out of bacteria is a virus infectious for bacteria. There are two phages: a phage which maintains a lysogen state for being integrated into a host's chromosome after invasion into bacteria and a destructive phage which maintains a lytic state for growing out of the chromosome and kills the host cells. The former is a λ phage infectious for Escherichia coli and the phage is incorporated into the chromosome by recombination of two specific homologous sequences of attP on a phage sequence and attB on a bacterial chromosome.

PCC6803 which is a strain of cyanobacteria retains a plurality of copied main chromosomes of 3.5 Mbp, and further has various plasmids such as pSYSM (120kbp), pSYSX (106kbp), pSYSA (103kbp), pSYSG (44kbp), pCC5.2 (5.2kbp), pCA2.4 (2.4kbp), and pCB2.4 (2.4kbp). The homologous coding regions of the ssb gene of Escherichia coli F factor are present on the PCC6803 chromosome and present on the pSYSA plasmid. The main chromosome and plasmid are rotary-replicated through the rolling cycle mechanism and they can be exchanged between cells by conjugation. This infers the transition and possibility of chromosome rearrangement. In recent years, the presence of a mobile insertion element on the PCC6803 genome has been revealed.

The unstable region on the present chromosome and the transition of the past chromosome rearrangement are revealed by investigating whether these transpositioned, moved, and parasitized molecules are currently present in cells, or whether the remaining of the molecules as a trace of the presence in the past is present, or whether phylogenetic properties are observed in the remaining of the molecules. The investigation of phylogenetic properties and structuralization of mobile molecules for different strains or types is expected to lead to the elucidation of biological evolution.

The present inventors conducted intensive studies focusing on homologous string pieces. As a result, they have arrived at the method of analyzing homologous domain phylogeny for characterizing the structure of the strings based on the phylogeny analysis of the string pieces. The method of analyzing homologous domain phylogeny to be explained later includes the steps of analyzing a target string, extracting a pair of sequences in a section (homologous section) linked by the relationship of homology (homological relationship), and analyzing the phylogeny of the string using the extraction result.

The use of the method of analyzing homologous domain phylogeny to be explained later allows for the estimation of the transition of chromosome rearrangement and the unstable region by general and global analysis of the structure of the whole genome, not focusing on the modification on a specific gene sequence.

The method of analyzing homologous domain phylogeny to be explained later can be used for not only the phylogeny analysis of gene sequences but also a method including the steps of extracting a part similar to general string array (corresponding to the homologous string piece) and analyzing the phylogeny of the similar part.

The following explanation will be made taking the phylogeny analysis of gene sequences as an example.

### (First embodiment)

### <Configuration of information processing apparatus>

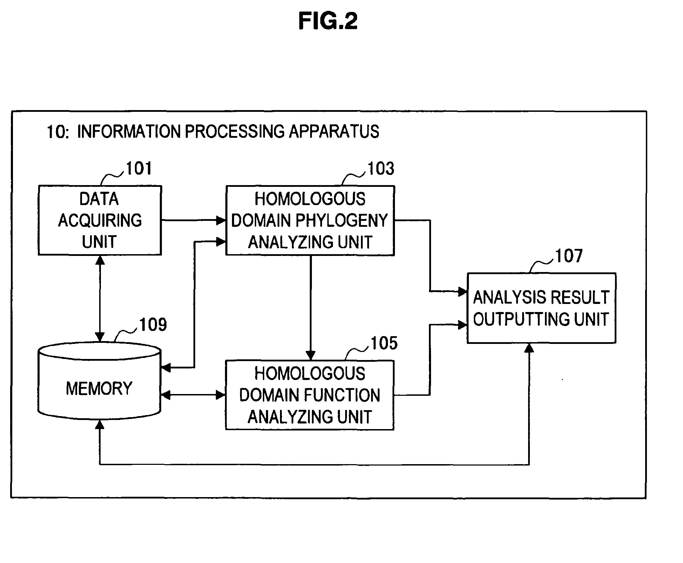

First, the configuration of the information processing apparatus according to the embodiment of the present invention will be described in detail with reference to FIG. 2. FIG. 2 is a block diagram showing the configuration of an information processing apparatus 10 according to the embodiment.

In the method of analyzing homologous domain phylogeny to be explained later, a target string is first aligned with itself and a pair of sequences in a section (homologous section) linked by the relationship of homology (homological relationship) is extracted. Then, in the analysis method, a bunch (homological group) in the homologous section linked by one or more homological relationship chains and a region (regional group) where homologous sections are regionally adjacent to or overlapped with each other is extracted and a combined group of the homological group and the regional group is defined as a family. Subsequently, in the analysis method, each family is subdivided according to the regional configuration of the homologous domain belonging to family to construct a phylogenetic tree. Hereinafter, the homologous section and homologous region are collectively referred to as a homologous domain.

An information processing apparatus 10 according to the present embodiment is an apparatus capable of executing the method of analyzing homologous domain phylogeny, and mainly includes a data acquiring unit 101, a homologous domain phylogeny analyzing unit 103, a homologous domain function analyzing unit 105, an analysis result outputting unit 107, and a memory 109 as shown in FIG. 2.

The data acquiring unit 101, for example, is realized by a CPU (Central Processing Unit), a ROM (Read Only Memory), a RAM (Random Access Memory), an input device, a communication device. The data acquiring unit 101 acquires string data to be targeted by the method of analyzing homologous domain phylogeny.

The data acquiring unit 101 may acquire the string data from various apparatuses connected via networks such as the Internet and a home network or may acquire the string data from various apparatuses directly connected to the information processing apparatus 10 via wired or wireless connection. The data acquiring unit 101 may use the data which is directly input to the information processing apparatus 10 by a user via various input devices such as a keyboard and a touch panel as the string data.

The data acquiring unit 101 outputs the acquired string data to the homologous domain phylogeny analyzing unit 103 to be described later. The data acquiring unit 101 may associate the acquired string data with the time entry related to the time when the string data is acquired to store it in the memory 109 to be described later.

The homologous domain phylogeny analyzing unit 103 is realized, for example, by the CPU, ROM, RAM, and communication device. The homologous domain phylogeny analyzing unit 103 analyzes the string data output from the data acquiring unit 101, extracts homologous string pieces in the string represented by the string data, and performs the phylogeny analysis of the extracted homologous string pieces.

When the phylogeny analysis processing based on the homologous domain is finished, the homologous domain phylogeny analyzing unit 103 outputs the obtained result to the homologous domain function analyzing unit 105 and the analysis result outputting unit 107 to be described later. The homologous domain phylogeny analyzing unit 103 may store the obtained analysis result in the memory 109 to be described later. When referring to the analysis result again, the analysis result can be easily acquired by storing the obtained analysis result, for example, in a form like a database. The database in which the obtained analysis result is recorded can be used for a process of new phylogeny analysis processing, so that the high efficiency of the analysis processing as well as the high accuracy of the analysis result can be achieved.

The detailed configuration and function of the homologous domain phylogeny analyzing unit 103 will be described below.

The homologous domain function analyzing unit 105 is realized, for example, by the CPU, ROM, RAM, and communication device. The homologous domain function analyzing unit 105 analyzes the function of the extracted homologous domain based on the analysis result by the homologous domain phylogeny analyzing unit 103.

When performing the homologous domain function analysis processing, the homologous domain function analyzing unit 105 can refer to various functional databases present on networks such as the Internet. Data about the homologous domain function (function data) are included in the analysis results output from the homologous domain phylogeny analyzing unit 103. An object being externally referenced as well as the ID and function ID in externally referencing data may be included in the function data. The homologous domain function analyzing unit 105 can search various functional databases present on the networks using such various IDs.

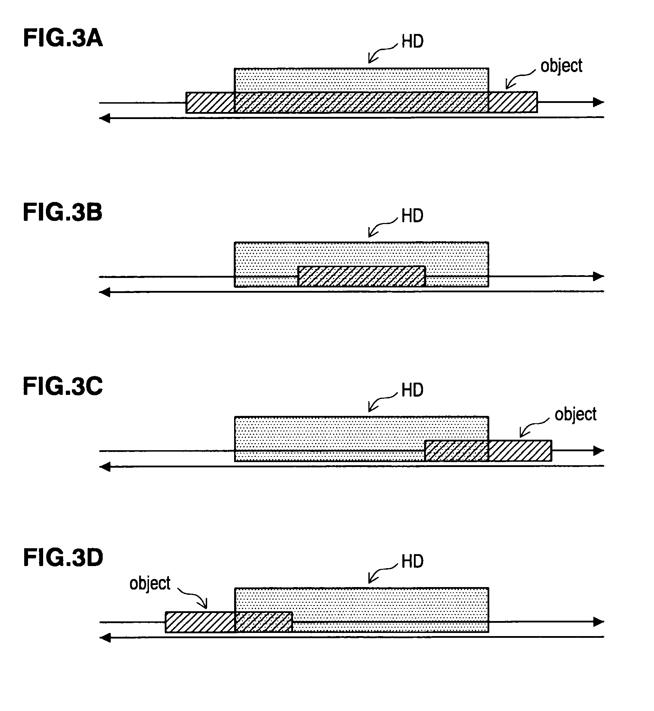

For example, as shown in FIG. 3, the case where an external reference object is present around a homologous domain HD belonging to the family being focused will be discussed. As shown in FIG. 3A, the external reference object may be an object related to a position over the wider range than the homologous domain HD being focused or may be an object related to a position over a range included in the homologous domain HD being focused as shown in FIG. 3B. As shown in FIGS. 3C and 3D, the external reference object may be an object in a position where a part of the object is overlapped with the homologous domain HD being focused.

The homologous domain function analyzing unit 105 is used to classify the function data collected in the whole family and estimate the function related to family. The function of the homologous domain extracted by the homologous domain phylogeny analyzing unit 103 can be estimated by performing such a process. This allows for deep interpretation of the analysis result obtained by the method of analyzing homologous domain phylogeny.

The homologous domain function analyzing unit 105 outputs the obtained result to the analysis result outputting unit 107 to be described later. The homologous domain function analyzing unit 105 may store the obtained analysis result in the memory 109 to be described later. When referring to the analysis result again, the analysis result can be easily acquired by storing the obtained analysis result, for example, in a form like a database. The database in which the obtained analysis result is recorded can be used for a process of new function analysis processing, so that the high efficiency of the analysis processing as well as the high accuracy of the analysis result can be achieved.

The analysis result outputting unit 107 is realized, for example, by the CPU, ROM, RAM, output device, and communication device. The analysis result outputting unit 107 outputs the analysis result obtained by analysis by the homologous domain phylogeny analyzing unit 103 and the analysis result obtained by analysis by the homologous domain function analyzing unit 105. In this case, the analysis result outputting unit 107 may visualize the various analysis results thus obtained to display them on an output device, such as a display, included in the information processing apparatus 10. The analysis result outputting unit 107 may output the various analysis results thus obtained via an output device such as a printer to use them as character information. The analysis result outputting unit 107 may output data showing the obtained analysis results to various removable recording media connected to the information processing apparatus 10 or an external connection equipment. The analysis result outputting unit 107 may output the data showing the obtained analysis results to an external network via the communication device.

When outputting the obtained analysis results, the analysis result outputting unit 107 can use known databases as well as various tools and programs.

The memory 109 is an example of a storage device included in the information processing apparatus 10 according to the present embodiment. String data being subjected to homologous domain phylogeny analysis processing and various analysis results obtained by the analysis may be stored in the memory 109. Various history information such as history information about the execution of homologous domain phylogeny analysis processing and history information about the acquisition of string data may be recorded in the memory 109. Various parameters saved when the information processing apparatus 10 according to the present embodiment performs any processing and the intermediate results during the processing, or various databases and programs are suitably recorded in the memory 109.

In the memory 109, each processing unit included in the information processing apparatus 10 according to the present embodiment is capable of reading and writing data freely.

### [Configuration of homologous domain phylogeny analyzing unit]

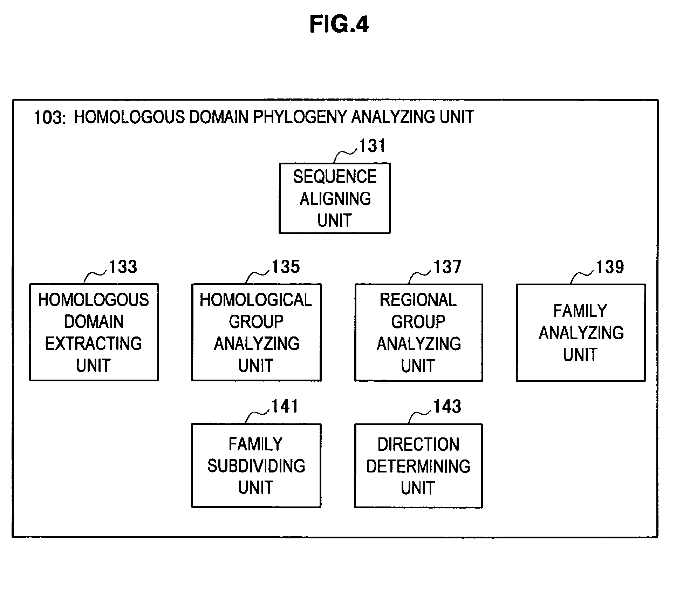

Subsequently, the configuration of the homologous domain phylogeny analyzing unit 103 according to the present embodiment will be described more in detail with reference to FIG. 4. FIG. 4 is a block diagram showing the configuration of the homologous domain phylogeny analyzing unit 103 according to the embodiment.

As shown in FIG. 4, the homologous domain phylogeny analyzing unit 103 further includes a sequence aligning unit 131, a homologous domain extracting unit 133, a homological group analyzing unit 135, a regional group analyzing unit 137, a family analyzing unit 139, a family subdividing unit 141, and a direction determining unit 143.

The sequence aligning unit 131 is realized, for example, by a CPU, ROM, and RAM. The sequence aligning unit 131 performs sequence alignment on a plurality of string data output from the data acquiring unit 101 and calculates a similarity measure between the string data (or a dissimilarity measure). More particularly, the sequence aligning unit 131 performs sequence alignment on all the combinations of two string data selected from the plurality of string data output from the data acquiring unit 101 based on known methods, and calculates the similarity measure (or the dissimilarity measure) between elements constituting respective string data (for example, numbers and alphabets constituting the string data).

Here, the sequence aligning unit 131 may calculate the similarity and score representing the degree of similarity between two string data as an amount representing the similarity measure. The sequence aligning unit 131 may calculate various distance metrics representing the degree of dissimilarity between two string data as an amount representing the dissimilarity measure.

For example, when calculating the distance metric between two string data, the sequence aligning unit 131 can calculate various distance metrics such as Hamming distance, Levenshtein distance, Smith-Waterman distance, and known distance metrics such as distance metrics using those distance metrics in combination with Entropy and N-gram analysis.

The sequence aligning unit 131 can use known calculation methods when calculating a score between two string data.

Here, each of the string data being focused is referred to as a module Mᵢ and a set {Mᵢ} (1≤i≤N) of the string data being focused is represented by a system G to be applied to the method of analyzing homologous domain phylogeny according to the present embodiment.

Each module Mᵢ includes an information Sᵢ about string sequences (sequence information) and an information Cᵢ about functions (function data).

When N pieces of modules Mᵢ are output from the data acquiring unit 101, the sequence aligning unit 131 according to the present embodiment aligns the combination of N type of Sᵢ vs. Sᵢ (namely, each to itself) and the combination of [{N(N-1)}/2] type of Sᵢ vs. Sⱼ (i≠j) based on the sequence data Sᵢ (1≤i≤N) for each module Mᵢ. In other words, the sequence aligning unit 131 performs sequence alignment on the N pieces of modules being focused in a round robin manner.

The information representing the sequence alignment result by the sequence aligning unit 131 is stored in the memory 109 and is used for the processing in each processing unit included in the homologous domain phylogeny analyzing unit 103.

The homologous domain extracting unit 133 is realized, for example, by the CPU, ROM, and RAM. The homologous domain extracting unit 133 extracts the information about homologous domains (homologous domain data) from string data being focused (module) using the sequence alignment result by the sequence aligning unit 131. Here, the homologous domain information includes an information Lᵢ about the homologous section (locus) representing the section of the homologous string piece (homologous section information) and an information Aₖ representing the homological relationship (align) between the information Lᵢ and a homologous section information Lⱼ as a pair thereof (homological relationship information).

The method of extracting the homologous section (hereinafter, simply referred to as a section) from the sequence alignment result is not particularly limited and various methods may be used. For example, the homologous domain extracting unit 133 refers to the information representing the sequence alignment result to extract a section whose value representing the similarity measure described (for example, a section whose similarity is equal to a predetermined threshold value or more, or the distance metric is equal to a predetermined threshold value or less) is more than a predetermined value as a homologous section.

Here, the homologous section information Lᵢ is the information represented by a combination of a start point s with an end point e of the homologous section on a module M to which the homologous section belongs. Hereinafter, the homologous section information Lᵢ is represented by Lᵢ = (M:s, M:e). A set of homologous section information is L = {Lᵢ}.

The homological relationship information Aₖ is the information represented by a combination of two homologous sections Lᵢ and Lⱼ, a directionality d (= 1 or -1), and a homology rate r (0<r≤1). Hereinafter, the homological relationship information Aₖ will be represented by Aₖ = (Lᵢ,Lⱼ,d,r). A set of homological relationship information is A = {Aₖ}.

The homologous domain information extracted by the homologous domain extracting unit 133 is stored in the memory 109 and is used for the processing in each processing unit included in the homologous domain phylogeny analyzing unit 103.

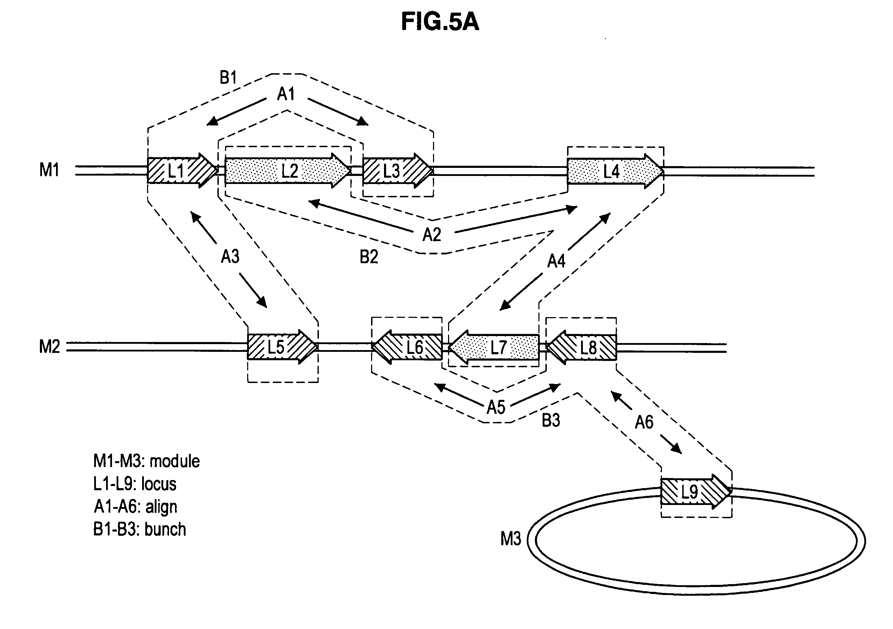

Specific examples of the homologous section and the homological relationship will be described with reference to FIG. 5A. FIG. 5A is an explanatory diagram for explaining homologous domain phylogeny analysis processing.

FIG. 5A shows the case where three modules M1 to M3 are present as modules to be used for the method of analyzing homologous domain phylogeny. Nine homologous sections L1 to L9 are extracted by sequence alignment on these modules M1 to M3. Here, the homologous sections L1 to L4 are present on the module M1, the homologous sections L5 to L8 are present on the module M2, and the homologous section L9 is present on the module M3.

Here, the homologous section L1 is linked with the homologous section L3 by the homological relationship A1 and the direction of the section L1 is the same as that of the section L3. Similarly, the homologous section L2 is linked with the homologous section L4 by the homological relationship A2. The direction of the section L2 is the same as that of the section L4. The homologous section L5 is linked with the homologous section L1 by the homological relationship A3 and the direction of the section L1 is the same as that of the section L5. The homologous section L4 is linked with the homologous section L7 by the homological relationship A4 and the direction of the section L4 is opposite to that of the section L7. The homologous section L6 is linked with the homologous section L8 by the homological relationship A5 and the direction of the section L6 is the same as that of the section L8. The homologous section L8 is linked with the homologous section L9 by the homological relationship A6 and the direction of the section L8 is opposite to that of the section L9.

The homologous domain extracting unit 133 is configured to analyze the processing result by the sequence aligning unit 131 to extract the above homologous domain information.

The homological group analyzing unit 135 is realized, for example, by the CPU, ROM, and RAM. The homological group analyzing unit 135 analyzes homologous domains (more particularly, the homological relationship information) dispersedly present in the same string data or between different string data to group domains having common homologous section information of the homologous domains into a homological group (bunch). Thus, when the homologous string pieces are scattered in the system G, a group of homologous objects (homological group) is lumped together as a "bunch".

Here, a bunch Bₕ is represented by a pair of a homologous section set {Lᵢ} and a homological relationship set {Aₖ} shared. Hence, Bₕ is ({Lᵢ}, {Aₖ}).

Specifically, the homological group analyzing unit 135 refers to each of the homological relationship information Aₖ (= (L1,L2,d,r)) to extract the information in which either the section L1 or the section L2 in the homological relationship information is present. Then, when neither the section L1 nor the section L2 belongs to any bunch, the homological group analyzing unit 135 registers ({L1,L2},{Aₖ}) as a new bunch. When the section L1 already belongs to a bunch Bₕ and the section L2 does not belong to the bunches, the homological group analyzing unit 135 additionally registers the homologous section L2 and the homological relationship Aₖ in the bunch Bₕ. When the sections L1 and L2 are opposite in a situation (namely, the section L2 already belongs to a bunch and the section L1 does not belong to the bunches), the same processing is performed. There is a case where the section L1 already belongs to a bunch B1 = ({Lᵢ},{Aₘ}) and the section L2 already belongs to a bunch B2 = ({Lⱼ},{Aₙ}). In this case, the homological group analyzing unit 135 combines the bunch B1 with the bunch B2, makes B1 = ({Lᵢ}∪{Lⱼ},{Aₘ}∪{Aₙ}), and deletes the bunch B2.

The information about homological group (bunch) formed by the homological group analyzing unit 135 is stored in the memory 109 and is used for the processing in each processing unit included in the homologous domain phylogeny analyzing unit 103.

In the case of the example shown in FIG. 5A, the homologous section L1 and the section L3 are linked by the homological relationship A1, and the homologous section L1 and the section L5 are linked by the homological relationship A3. Accordingly, the two homological relationships share the homologous section L1. Therefore, the homologous sections L1, L3, and L5 form the bunch B1. Similarly, the homologous section L2 and the section L4 are linked by the homological relationship A2, and the homologous section L4 and the section L7 are linked by the homological relationship A4. Accordingly, the two homological relationships share the homologous section L4. Therefore, the homologous sections L2, L4, and L7 form the bunch B2. The homologous section L6 and the section L8 are linked by the homological relationship A5, and the homologous section L8 and the section L9 are linked by the homological relationship A6. Accordingly, the two homological relationships share the homologous section L8. Therefore, the homologous sections L6, L8, and L9 form the bunch B3.

The regional group analyzing unit 137 is realized, for example, by a CPU, ROM, and RAM. The regional group analyzing unit 137 analyzes homologous domains dispersedly present in the same string data or between different string data to produce a regional group representing inclusion and overlap relationships between the homologous domains.

Despite the fact that almost the same homologous section in implied in the sequence alignment result, there may be slightly different aligned regions depending on their aligned counterpart regions. Thus, homologous sections having a plurality of different lengths in a state where a few more bases are present or a much fewer bases are present at the start or end points are detected in some cases. In order to combine together, a plurality of the homologous sections whose end is slightly different, the concept of a region in which margins are added to the ends of the homologous section is introduced.

In order to represent the inclusion and overlap relationships between regions, a region including the homologous section and a predetermined length of margin section which is added to both ends of the homologous section is set as a small region (region) in the present embodiment. In the present embodiment, a small region which is included in neither of the other small regions is defined as a middle region (ceiling), and a set of the middle regions overlapped is set as a large region (domain). Each of the regions is collectively referred to as a regional group.

The regional group analyzing unit 137 first produces a small region (region) Rᵢ using a start midpoint s, an end midpoint e, a margin ratio m, and a set L of the homologous section belonging to the small region. Hereinafter, the region Rᵢ is represented by Rᵢ = (s,e,m,{Lᵢ}). The regional group analyzing unit 137 determines λ = (λ_{R}×m)/100 as a margin length based on λ_{R} = |e-s+1| that is the total length of the middle section of the small region, determines a section represented by [s-λ,s+λ] as an effective starting section, and determines a section represented by [e-λ,e+λ] as an effective ending section. Here, the margin length is shown when the margin ratio is given in percentage, however the margin length is represented by λ = λ_{R}×m when the margin ratio is represented in decimal.

The regional group analyzing unit 137 suitably sets a value of the margin ratio m. The set value may be a value acquired as user setting information at the start of homologous domain phylogeny analysis processing or may be a set value set in advance. The regional group analyzing unit 137 may dynamically change the value of the margin ratio m.

Subsequently, for each homologous section Lᵢ= (sᵢ,eᵢ), the regional group analyzing unit 137 determined whether a small region is present, whose effective starting section includes the start point of the homologous section Lᵢ, and whose effective ending section includes the end point of the homologous section Lᵢ. When the small region satisfying the conditions is present, the regional group analyzing unit 137 adds the homologous section Lᵢ being focused to the homologous section set of the detected small region. On the other hand, when the small region satisfying the conditions is not present, the regional group analyzing unit 137 determines the start point of the homologous section being focused as a start midpoint, determines the end point of the homologous section as an end midpoint, and registers (sᵢ,eᵢ,m,{Lᵢ}) as a new small region.

The regional group analyzing unit 137 repeats the above process, so that it can specify one small region to which each of the homologous sections Lᵢ belongs for each of the homologous sections Lᵢ.

The regional group analyzing unit 137 refers to values such as the effective starting section [s-λ,s+λ] and the effective ending section [e-λ,e+λ] for each of the small regions Rᵢ to analyze the inclusion and overlap relationships between the small regions. As a result of the analysis, when small regions in the inclusion relationship and small regions with overlapping regions are present, the regional group analyzing unit 137 sets to make related small regions referred to each other.

Subsequently, the regional group analyzing unit 137 determines whether a small region is present for each of the small regions Rᵢ which is not included in either of the other small regions. When such a small region is present, the regional group analyzing unit 137 sets the small region which is not included in either of the other small regions as a middle region (ceiling) Cᵢ.

The regional group analyzing unit 137 determines whether a mutually overlapping region are present for each of the middle regions Cᵢ. When a mutually overlapping middle region is present, the regional group analyzing unit 137 sets a set of the middle regions Cᵢ as a large region (domain) Dₕ = {Cᵢ}.

Here, two large regions Dₕ and Dₖ (h≠k) are not overlapped with each other. One small region belongs is one or more middle regions, however there is only one large region to which one small region belongs.

The regional group analyzing unit 137 performs the above process, so that it can produce the regional group representing inclusion and overlap relationships between the homologous domains. The information about the regional group produced by the regional group analyzing unit 137 is stored in the memory 109 and is used for the processing in each processing unit included in the homologous domain phylogeny analyzing unit 103.

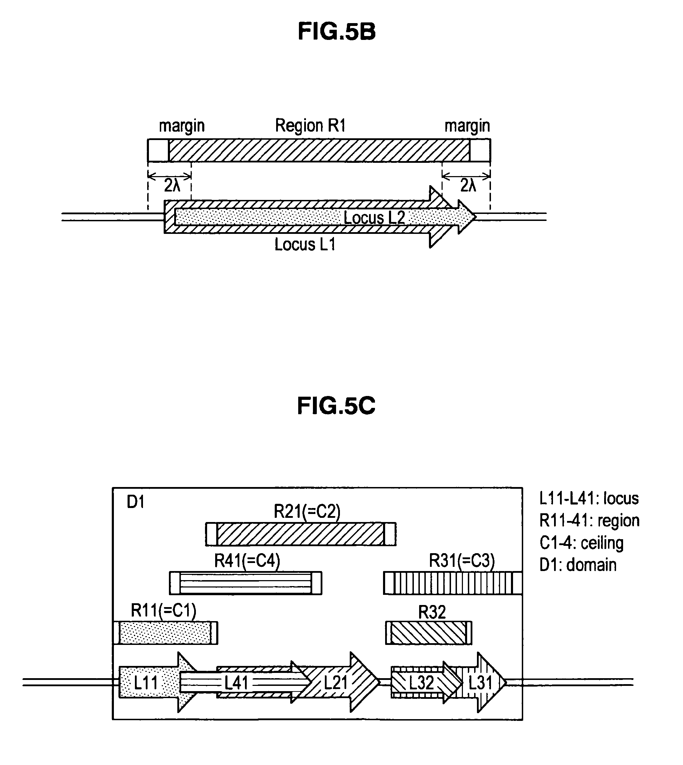

Hereinafter, a relationship between homologous sections and a small region will be specifically explained with reference to FIG. 5B. FIG. 5B is an explanatory diagram for explaining homologous domain phylogeny analysis processing.

A small region R1 shown in FIG. 5B is a region including a region corresponding to the homologous section L1 and margin sections provided at both ends of the region corresponding to the homologous section L1 as illustrated in the drawing. Here, the region indicated by a white rectangle provided at the left side of the homologous section L1 is a region represented by [s-λ,s], and the region indicated by a white rectangle provided at the right side of the homologous section L1 is a region represented by [e,e+λ]. Therefore, the effective starting section [s-λ,s+λ] ranges from the left end of the small region R1 to 2λ rightward as shown in FIG. 5B. Similarly, the effective ending section [e-λ,e+λ] ranges from the right end of the small region R1 to 2λ leftward as shown in FIG. 5B.

Aside from the homologous section L1, the case where the homologous section L2 is detected as illustrated in the drawing will be discussed. As is apparent from FIG. 5B, the start point of the homologous section L2 is positioned within the effective starting section and the end point of the homologous section L2 is positioned within the effective ending section. Therefore, the regional group analyzing unit 137 determines that the homologous section L1 and the homologous section L2 belong to the same small region R1.

Subsequently, relationships among small, middle, and large regions will be specifically explained with reference to FIG. 5C. FIG. 5C is an explanatory diagram for explaining homologous domain phylogeny analysis processing.

The example shown in FIG. 5C shows the case where five homologous sections L11, L21, L31, L32, and L41 are present on a certain module M. In this case, the regional group analyzing unit 137 adds the margin sections explained above to both ends of respective homologous sections and sets the five small regions R11, R21, R31, R32, and R41 as illustrated in the drawing. As is apparent from FIG. 5C, the small regions R11, R21, R31, and R41 are not included in the other small regions. Therefore, the four small regions are set as middle regions C1, C2, C3, and C4, respectively. On the other hand, the small region R32 is included in the small region R31 as is clear from the drawing. Therefore, the small region R32 is not recognized as the middle region. When focusing on the ranges of the middle regions C1, C2, C3, and C4, a part of the middle region C1 is overlapped with the middle regions C2 and C4, and a part of the middle region C3 is overlapped with the middle region C2. Accordingly, the regional group analyzing unit 137 sets a set of the middle regions C1, C2, C3, and C4 as a large region D1.

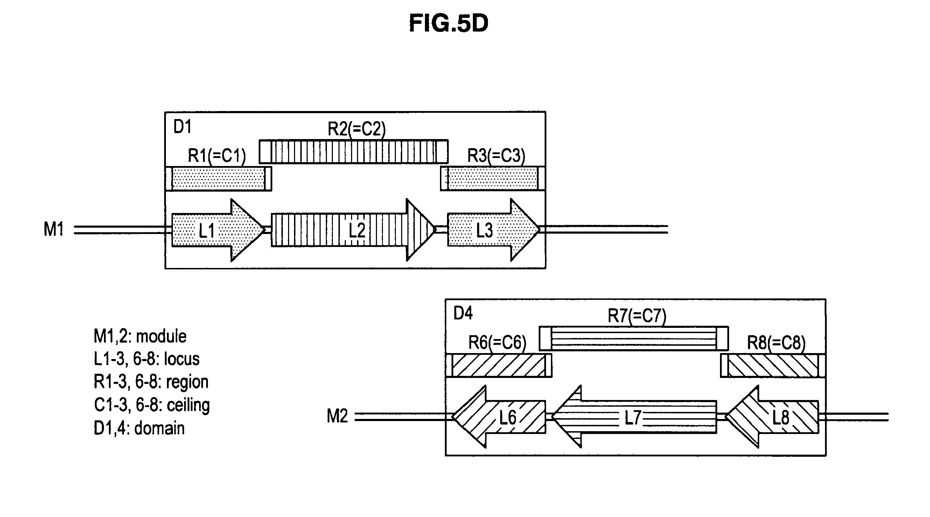

As shown in FIG. 5D, the homologous sections L1, L2, and L3 present on the module M1 in the example shown in FIG. 5A are recognized as the small regions R1, R2, and R3 including the margin sections by the regional group analyzing unit 137 and the large region D1 is formed from the overlap relationship. Similarly, the homologous sections L6, L7, and L8 present on the module M2 are recognized as the small region R6, R7, and R8 including the margin sections by the regional group analyzing unit 137 and a large region D4 is formed from the overlap relationship.

The family analyzing unit 139 is realized, for example, by the CPU, ROM, and RAM. The family analyzing unit 139 analyzes a family of the homologous section based on the information about the homological group produced by the homological group analyzing unit 135 and the information about the regional group produced by the regional group analyzing unit 137.

Pieces B and C in different places in a certain string A as well as their homologous pieces B' and C' are scattered in the system. Here, the original pieces B and C of the pieces B' and C' where no homological relationship exists constitute the string A, between the pieces B' and C'. It is the concept of a family in the method of analyzing homologous domain phylogeny according to the present embodiment that the pieces B' and C' have either been derived from the string A accounting for one area or constructed the string A. Therefore, the family analyzing unit 139 according to the present embodiment specifies that one family consists of homological groups and regional groups. Although the derivation and the construction mean oppositely ordered phenomena, the homological relationship is treated as a relationship without directionality in the method of analyzing homologous domain phylogeny according to the present embodiment. Thus, any description about either the derivation or construction will be avoided.

As explained above, the family analyzing unit 139 treats a combined group of a homological group B = {Bᵢ} and a regional group X = {Xⱼ} as one family BXFᵢ = (B,X). The family analyzing unit 139 first focuses on a bunch set {Bᵢ} to which each of the sections in the homologous section set {Lᵢ} belonging to each of the small regions Rᵢ belong and searches for the family to which any of the bunches belong. When such a family is detected, the family analyzing unit 139 additionally adds the small region R to a regional group R of a family BRFᵢ = (B,R). When such a family is not detected, the family analyzing unit 139 registers a family BRFᵢ = ({Bᵢ},{Rᵢ}) having the bunch set {Bᵢ} of the small regions Rᵢ as a regional group and {Rᵢ} as a regional group as a new family.

Similarly, the family analyzing unit 139 analyzes the family BCFᵢ in all the middle regions Cᵢ and the family BDFᵢ in all the large regions Dᵢ.

The family analyzing unit 139 performs the above process, so that it can produce a family including the homological group and the regional group. The information about each family produced by the family analyzing unit 139 is stored in the memory 109 and is used for the processing in each processing unit included in the homologous domain phylogeny analyzing unit 103.

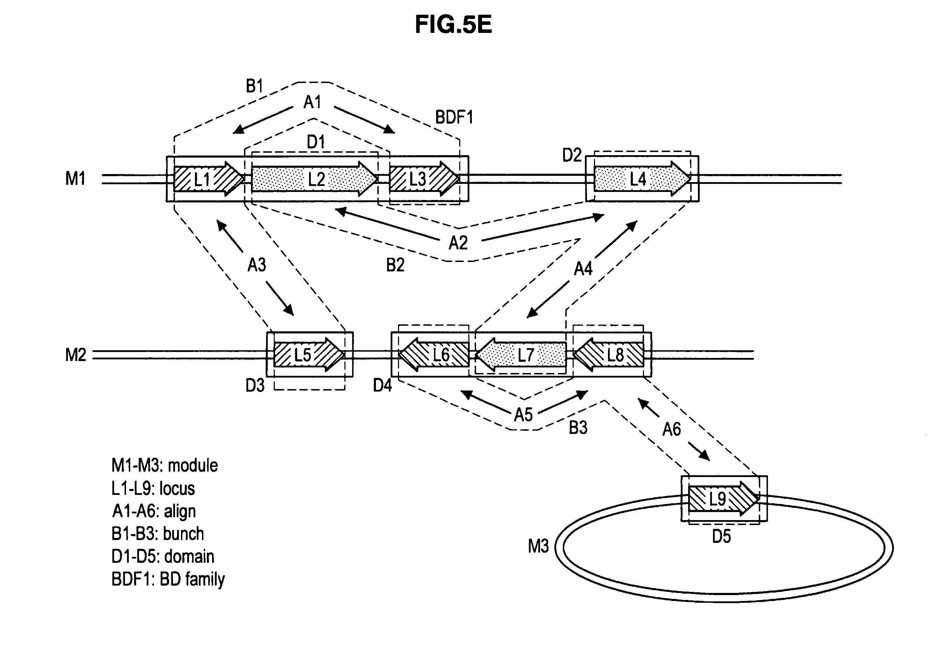

Subsequently, the family according to the present embodiment will be specifically explained with reference to FIG. 5E. FIG. 5E is an explanatory diagram for explaining homologous domain phylogeny analysis processing.

As shown in FIG. 5E, the homologous sections L1 and L3 belonging to the bunch B1 and the homologous section L2 belonging to the bunch B3 in the example shown in FIG. 5A form the large region D1 from the geographically adjacent relationship. The homologous section L7 belonging to the bunch B2 and the homologous sections L6 and L8 belonging to the bunch B3 similarly form the large region D4 from the geographically adjacent relationship. Therefore, one large family BDF1 = ({B1,B2,B3},{D1,D2,D3, D4,D5}) is formed from the bunches B1, B2, and B3 and the large region belonging thereto.

As with the large region, the small and middle regions also form families. When the small regions of the homologous sections L1 to L9 are defined as R1 to R9 and the middle regions are defined as C1 to C9, three families BRF1 = ({B1},{R1,R3,R5}), BRF2 = ({B2}, {R2,R4,R7}), and BRF3 = ({B3},{R6,R8,R9}) are present as small families. Similarly, three families BCF1 = ({B1},{C1,C3,C5}), BCF2 = ({B2},{C2,C4,C7}), and BCF3 = ({B3},{C6,C8,C9}) are present as middle families.

The family subdividing unit 141 is realized, for example, by the CPU, ROM, and RAM. The family subdividing unit 141 respectively subdivides the large region families and the middle region families produced by the family analyzing unit 139 to produce large region sub-families and large region sub-sub-families.

According to the concept of the above families, when homologous pieces in a certain string are scattered, all the homologous domains regionally adjacent to each other are included in to one large family. In this case, it is considered that the geographically adjacent relationship is weak compared with the homological relationship in the method of analyzing homologous domain phylogeny according to the present embodiment. Thus, the large region family is subdivided based on the hierarchy of the regional structure.

A set {BCFᵢ} of the middle region families of the middle regions {Cⱼ} constituting each of the large regions Dᵢ belonging to the large region family BDFᵢ = ({B},{D}) is a union of the subsets of the large region family. When one or more large regions share a subset of the middle region families, the family subdividing unit 141 defines the subset of the middle region families as a large region sub-family BDCFᵢ = ({BCFᵢ},{Dᵢ}).

The family subdividing unit 141 searches for the large region sub-family as follows.
For each large region, the family subdividing unit 141 first determines a set of middle regions which forms the large region, and searches for a sub-family BDCFᵢ matching a set of middle region families to which each of the middle regions belong. When such a sub-family is detected, the family subdividing unit 141 additionally adds the large region to the sub-family detected. When such a sub-family is not detected, the family subdividing unit 141 newly registers the large region sub-family BDCFᵢ = ({BCFᵢ},{Dᵢ}) including {Dᵢ} as a regional group.

Similarly, a set {BRFᵢ} of the small region families of the small regions {Rⱼ} included in each of the middle regions Cᵢ belonging to the middle region family BCFᵢ = ({B},{C}) is a union of the subsets of the middle region family. When one or more middle regions share a subset of the small region families, the family subdividing unit 141 defines the subset of the small region families as a large region sub-sub-family BDCRFᵢ = ({BRFᵢ},{Dᵢ}).

The family subdividing unit 141 searches for the large region sub-sub-family as follows.
For each large region sub-family BDCFᵢ, the family subdividing unit 141 first determines a set of large regions which belongs to the large region sub-family, a set of middle regions which forms the large region, a set of small regions included in the middle regions, and searches for a large region sub-sub-family BDCRFᵢ matching a set of small region families BRFᵢ to which each of the small regions belong. When such a sub-sub-family is detected, the family subdividing unit 141 additionally adds the large region to the detected sub-sub-family. When such a sub-sub-family is not detected, the family subdividing unit 141 newly registers the large region sub-sub-family BDCRFᵢ = ({BRFᵢ},{Dᵢ}) including {Dᵢ} as a regional group.

The family subdividing unit 141 performs the above process, so that it can hierarchically subdivide the large region family. The information about the sub-family and the sub-sub-family produced by the family subdividing unit 141 is stored in the memory 109 and is used for the processing in each processing unit included in the homologous domain phylogeny analyzing unit 103.

In the example shown in FIG. 5E, as for a large family BDF1 = ({B1,B2,B3},{D1,D2,D3,D4,D5}), the following five large region sub-families are detected by the family subdividing unit 141.

Large region sub-family
BDCF11 = ({BCF1,BCF2},{D1})
BDCF12 = ({BCF2},{D2})
BDCF13 = ({BCF1},{D3})
BDCF14 = ({BCF2,BCF3},{D4})
BDCF15 = ({BCF3},{D5})

The following five large region sub-sub-families are detected by the family subdividing unit 141.

Large region sub-sub-family
BDCRF111 = ({BRF1,BRF2},{D1})
BDCRF121 = ({BRF2},{D2})
BDCRF131 = ({BRF1},{D3})
BDCRF141 = ({BRF2,BRF3},{D4})
BDCRF151 = ({BRF3},{D5})

The direction determining unit 143 is realized, for example, by the CPU, ROM, and RAM.
For example, DNA is formed of a double strandreadable in a reverse complemental manner. Taking into consideration of a common palindrome, including the possibility of reverse reading, it is preferable to determine a standard direction of an element so that the element in which the homologous section, the small region, the middle region, and the large region are linked by the bunch and belonging to the same family is referred to in the consistent alignment direction.

Then, the direction determining unit 143 determines the alignment direction (standard direction) of the section of the homologous string piece which belongs to the same homological group and the same family among the sections of homologous string pieces belonging to the small, middle, and large regions. The direction determining unit 143 determines what order to determine the direction of the homologous sections on the bunch by a heuristic method based on the following priorities.

When the direction of any of the homologous sections present on the bunch is not determined yet, the direction determining unit 143 determines the alignment direction by the following procedure.

(a) When the direction of the regional group (small, middle, and large regions) to which the homologous section being focused belongs is determined, the direction determining unit 143 determines the direction of the region as the direction of the homologous section.
(b) When a gene (encoded region) is present around the homologous section being focused (the inside, outside, or ends of the homologous section), the direction determining unit 143 determines the direction of the homologous domain in accordance with the direction of the gene (encoded region).
(c) The direction determining unit 143 determines the direction of a homologous section which has the largest number of homological relationships and is estimated to be the center of a bunch as a normal direction (directionality d = 1).

The direction determining unit 143 determines the direction of a first homologous section on the bunch and transmits the determined direction to the regions (small, middle, and large regions) to which the homologous section with the direction determined belongs. The direction determining unit 143 transmits the determined direction to other homologous sections on the bunch.

The direction determining unit 143 can determine the alignment direction of the homologous section by performing such a process.

As explained above, the method of analyzing homologous domain phylogeny according to the present embodiment includes the steps of focusing on the homologous domain obtained from the alignment of the string and combining a homological group which is a group of homologous domains dispersed physically with a regional group which is a group of homologous domains geographically adjacent to form a family. Then, the obtained family is subdivided from the regional configuration and the string being focused is classified.

In other words, the method of analyzing homologous domain phylogeny is a method including the steps of focusing on a similar string piece called the homologous domain and estimating from the geographical structure the process that the homologous domains dispersed geographically have been transmitted while being structured or fragmented. Eventually, it is also a method including a step of characterizing the similarity between the string data being focused from the aspect of the occurrence pattern and phylogeny of similar string pieces appearing on the strings. For example, in the music field, the melody repeatedly appearing in a musical piece makes the musical piece impressive, or a theme of the whole musical piece is expressed by making an arranged form of a certain melody appeared in each movement. The use of the analysis method allows for the accurate expression and quantification of the user's subjective and intuitive impression on the data such that "these data seem to be similar".

Each of the processing units explained above perform each of the processes, so that the homologous domain phylogeny analyzing unit 103 according to the present embodiment can analyze the phylogeny of the homologous domain by focusing on the distribution and structure of string pieces similar to each other (namely, the homologous domain).

As described above, the configuration and function of the homologous domain phylogeny analyzing unit 103 according to the present embodiment have been explained in detail with reference to FIGS. 4 to 5E.

As described above, one example of the function of the information processing apparatus 10 according to the present embodiment has been described. Each of the constituent elements may be configured by using a general-purpose part or circuit, or it may be configured by hardware specialized to the function of each constituent element. Further, the function of each constituent element may be entirely realized by a CPU or the like. It is thereby possible to appropriately change the configuration to use according to the technique level available when implementing the present embodiment.

It is possible to make a computer program for realizing the functions of the above-described information processing apparatus according to the present embodiment, and the computer program can be implemented on a personal computer and the like. Further, a computer-readable recording medium storing such computer program can be provided. Examples of the recording medium include a magnetic disk, an optical disk, a magneto-optical disk, and a flash memory. Further, the above computer program may be distributed by, for example, a network, without using the recording medium.

The case where the information processing apparatus 10 according to the present embodiment includes the homologous domain function analyzing unit 105 has been described above, however the invention is not particularly limited to the example. The information processing apparatus 10 does not have to include the homologous domain function analyzing unit 105.

### <Flow of method of analyzing homologous domain phylogeny>

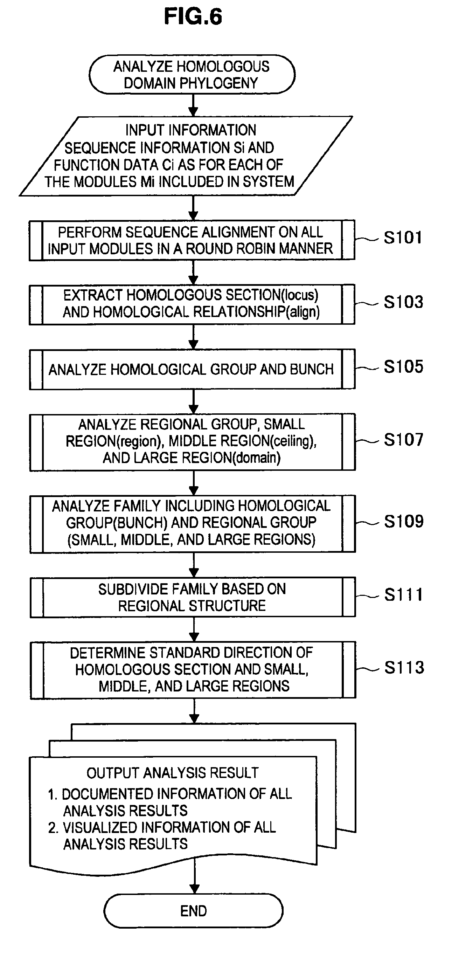

Subsequently, the flow of the method of analyzing homologous domain phylogeny to according to the present embodiment will be described with reference to FIG. 6. FIG. 6 is a flow chart showing the flow of the method of analyzing homologous domain phylogeny according to the present embodiment.

Before the following description, given a system including strings (modules) to be subjected to the method of analyzing homologous domain phylogeny, a sequence information Sᵢ and a function data Cᵢ as for each of the modules Mᵢ included in the system are input to the homologous domain phylogeny analyzing unit 103 from the data acquiring unit 101. Here, a parameter i is a parameter (where 1≤i≤N) representing the number of modules constituting a system.

First, the sequence aligning unit 131 of the homologous domain phylogeny analyzing unit 103 performs sequence alignment on all of the input modules in a round robin manner (step S101). Thus, the sequence aligning unit 131 produces the sequence alignment result of N type of each module to itself (sequence alignment result of Sᵢ vs. Sᵢ) and the sequence alignment result of {N(N-1)/2} type of Sᵢ vs. Sⱼ (i≠j).

Subsequently, the homologous domain extracting unit 133 extracts a string piece corresponding to the homologous section and its homological relationship using the sequence alignment result by the sequence aligning unit 131 (step S103). As a result, the homologous domain information including the homologous section information and the homological relationship information is produced.

Then, the homological group analyzing unit 135 analyzes the homologous domain information produced by the homologous domain extracting unit 133 to produce the information about the homological group (bunch) (step S105).

The regional group analyzing unit 137 analyzes the homologous domain information produced by the homologous domain extracting unit 133 to determine the regional group (step S107). As a result, a group of homologous domains geographically adjacent, such as small, middle, and large regions is produced.

Subsequently, the family analyzing unit 139 analyzes the homological group produced by the homological group analyzing unit 135 and the regional group produced by the regional group analyzing unit 137 to determine the family of the homologous domain (step S109). Thus, the family analyzing unit 139 produces information about the large region family, middle region family, and small region family.

Thereafter, the family subdividing unit 141 subdivides the large region family and middle region family produced by the family analyzing unit 139 based on the regional structure (step S111). As a result, the large region sub-family in which the large region family is subdivided and the large region sub-sub-family in which the large region family is further subdivided are produced.

Subsequently, the direction determining unit 143 determines a standard direction of an element so that the element in which the homologous section, the small region, the middle region, and the large region are linked by the bunch and belonging to the same family is referred to in the consistent alignment direction (step S113).

After the above-described process, the homologous domain phylogeny analyzing unit 103 outputs the obtained analysis result to the analysis result outputting unit 107. The analysis result outputting unit 107 outputs the analysis result by the homologous domain phylogeny analyzing unit 103 via various output devices, thereby allowing the analysis result to be notified to the user. Here, the analysis result output by the analysis result outputting unit 107 includes, for example, documented information of all analysis results and visualized information of all analysis results as shown in FIG. 6.

### <Flow of method of analyzing homologous domain function>

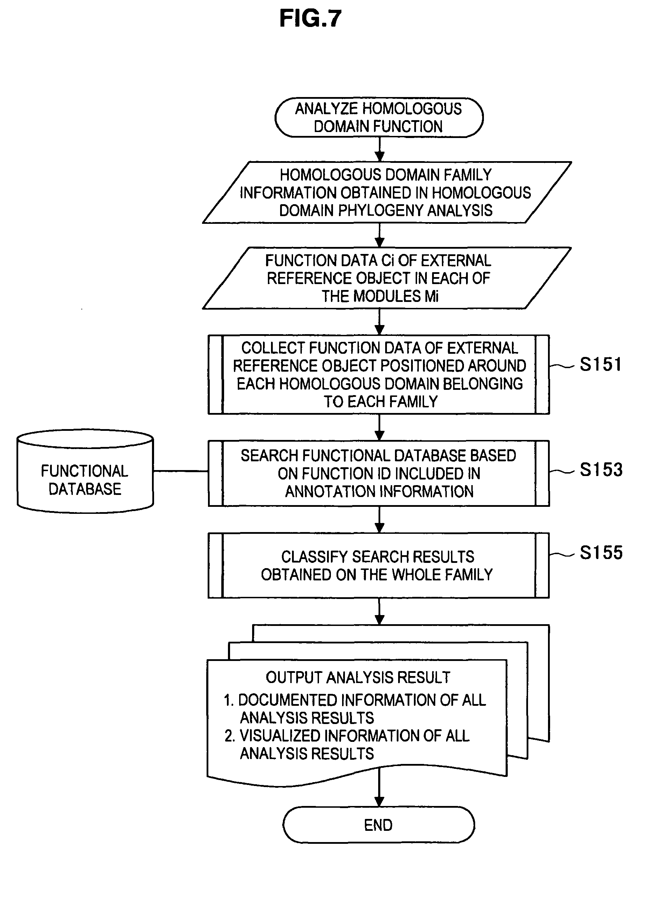

Subsequently, the flow of the method of analyzing homologous domain function according to the present embodiment will be described with reference to FIG. 7. FIG. 7 is a flow chart showing the flow of the method of analyzing homologous domain function according to the present embodiment.

Before the following description, the information about the family of the homologous domain obtained by the homologous domain phylogeny analysis (homologous domain family information) and the function data Cᵢ of the external reference object in each of the modules Mᵢ being focused are input to the homologous domain function analyzing unit 105.

The homologous domain function analyzing unit 105 collects the function data of the external reference object located around each homologous domain belonging to each family (step S151). Then, the homologous domain function analyzing unit 105 refers to annotation information included in the collected function data of the external reference object and searches the functional database based on the function ID included in the annotation information (step S153).

The functional database to be referred to by the homologous domain function analyzing unit 105 may be provided in the information processing apparatus 10 according to the present embodiment or may be a database managed by various servers connected to communications networks such as the Internet.

Then, the homologous domain function analyzing unit 105 classifies the search results obtained on the whole family (step S155). By performing the above-described processing, the homologous domain function analyzing unit 105 can estimate a function to be considered as the function of the homologous domain included in the family using the functions of external element functions as a means.

After the above-described process, the homologous domain function analyzing unit 105 outputs the obtained analysis result to the analysis result outputting unit 107. The analysis result outputting unit 107 outputs the analysis result by the homologous domain function analyzing unit 105 via various output devices, thereby allowing the analysis result to be notified to the user. Here, the analysis result output by the analysis result outputting unit 107 includes, for example, documented information of all analysis results and visualized information of all analysis results as shown in FIG. 7.

### <One example of software for analyzing genome structure>

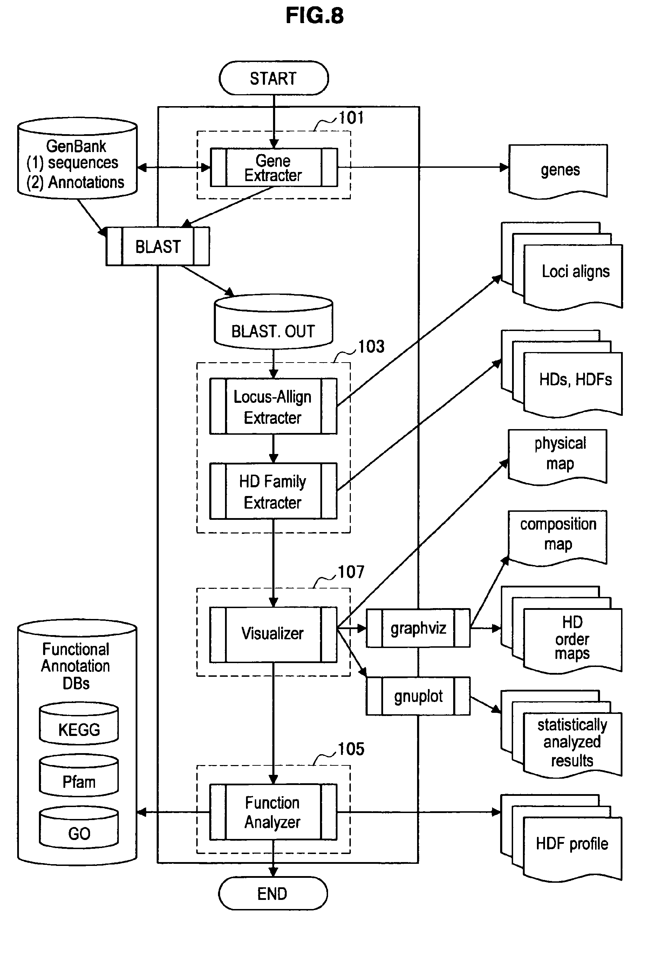

A software for analyzing genome structure was developed using the method of analyzing homologous domain phylogeny as described above. Hereinafter, the configuration of the developed software will be first described with reference to FIG. 8. FIG. 8 is an explanatory diagram showing an example of software for analyzing genome structure using the homologous domain phylogeny analysis processing and the homologous domain function analysis processing according to the present embodiment.

As shown in FIG. 8, the developed software for analyzing genome structure uses various existing databases. Further, existing tools are used to display the analysis result. The configuration of the software for analyzing genome structure to be described hereinafter is absolutely one example. The software for analyzing genome structure using the method of analyzing homologous domain phylogeny according to the present embodiment is not limited to the following examples.

Here, a module "Gene Extracter" in FIG. 8 corresponds to the data acquiring unit 101 of the information processing apparatus 10 according to the present embodiment and acquires various input information such as base sequence information and gene related information to be analyzed. Further, a module "Locus-Allign Extracter" in FIG. 8 extracts the homologous section and homological relationship and a module "HD Family Ectracter" analyzes the family of the base sequence being focused. The two modules correspond to the homologous domain phylogeny analyzing unit 103 of the information processing apparatus 10 according to the present embodiment. A module "Visualizer" in FIG. 8 corresponds to the analysis result outputting unit 107 according to the present embodiment, and a module "Function Analyzer" corresponds to the homologous domain function analyzing unit 105 according to the present embodiment.

In the software for analyzing genome structure according to the present embodiment, the following information is used as input information.

### Input information

(1) Ascii files containing nucleic acid sequence information with annotation of genes and encoding regions of chromosomes (or plasmids) to be analyzed based on a gb form of the gene information database: Genbank (http://www.ncbi.nlm.nih.gov/Genbank/), published by the National Center for Biotechnology Information (NCBI) belonging to the National Institutes of Health (NIH).
(2) Ascii files containing homologous sequence information output from the public software for alignment analysis of nucleic acid and amino acid sequences: BLAST (http://www.ncbi.nlm.nih.gov/blast/Blast.cgi) provided by the NCBI.
(3) Argument representing the margin ratio of small region (margin: margin length/total length × 100) (%) (MARGIN = Numerical value).
(4) Argument representing a debug mode switch (if DEBUG is 1, the debug mode is turned on; if DEBUG is 0 or not specified, the debug mode is turned off).

In the software for analyzing genome structure according to the present embodiment, the following information is used as output information.

### Output information

(1) Text (Ascii) file containing all analysis information including the position and function of genes (encoding regions), the position of homologous domain, phylogeny, distribution, surrounding gene clusters, related function, and statistical information
(2) Linear physical (or scaled) map in which the position and direction of genes of chromosomes (encoding regions) and the position, direction, and phylogeny of homologous domain are visualized by color (an SVG language file and an html language file used for invoking the SVG language file (Note 1)
(3) Scheme diagram in which the phylogeny of an internal structure of the homologous domain is visualized by color (The intermediate output is a DOT language file; The final output is a gif/jpg file produced with the public tool Graphviz) (Note 2) (4) Scheme diagram in which the phylogenetic tree of the homologous domain is visualized (The intermediate output is a DOT language file; The final output is a gif/jpg file produced with Graphviz)
(5) Scheme diagram in which homologous domains on gene molecules containing chromosomes are automatically aligned and visualized by color based on the order and phylogeny (The intermediate output is a DOT language file; The final output is a gif/jpg file produced with Graphviz) (Note 3)
(6) Graph in which statistical information about the length and distribution of the homologous domain belonging to the homologous domain family is visualized (The final output is a ps/pdf file provided with gnuplot which is a part of the GNU environment)

### (Note 1)

When directly outputting visualized information such as a physical distribution map, including a distribution map of gene homologous domains, the visualized information is output as a Scalable Vector Graphics (SVG) language file from the viewpoint of generality, high functionality, and expandability. An SVG language is a general-purpose language developed to describe two-dimensional graphics in an XML language form which is a standard language used to produce a Web and allows for not only visualization but also input function. The specifications are available from the World Wide Web Consortium (W3C)(2003). The SVG language file can be visualized using the known software. Further, it can be visualized by incorporating a predetermined plug-in for SVG interpretation to an Internet browser compatible with XML. For the latter, the SVG language file with the object as well as the html language file used for starting are output.

### (Note 2)

Visualized information including not only a physical distance but a relational distance, such as a scheme diagram of the internal structure of the homologous domain, a homologous domain family tree, and a homologous domain permutation diagram is first intermediately output as a DOT language file (Gansner et al. 2006) and automatically laid out with the known public software Graphviz. Thereafter, a gif (or jpg) file is obtained as a final output. The Graphviz software has five different layout algorithms: dot (for directed graphs), neato (for undirected graphs), twopi (for radial layouts), circo (for circular layouts), and fdp (for undirected hierarchy graphs). The scheme diagram of the internal structure of the homologous domain is automatically laid out with the fdp algorithm, the homologous domain family tree with the neato algorithm, and the homologous domain permutation diagram with the dot algorithm.

### (Note 3)

In the visualization process, individual families of the small, middle, and large regions are colored automatically. Hexadecimal number expression of a color circle (RGB) is (#000000-#FFFFFF). The color of the i-th family of N families is set to be # FFFFFF×i/(N+1).

Hereinafter, an example of execution environment of the software for analyzing genome structure according to the present embodiment will be described. The following execution environment is absolutely one example. The execution environment of the software for analyzing genome structure according to the present embodiment is not limited to the following examples.

### Execution environment

(1) A program is configured to run under the execution environment of Linux/Unix with GNU.
(2) The analysis program was described using a gawk script language. The gawk language processor is a script language embedded as a part of GNU.
(3) It is configured so that the software execution starts from a shell script used for starting.
(4) In the analysis of published gene sequences, the nucleic acid database: GenBank, provided by the NCBI affiliated with the National Institutes of Health is used.
(5) The public software for alignment analysis of nucleic acid and amino acid sequences: BLAST provided by the NCBI is used to extract homologous sequences. In this case, the Web server of NCBI may be used.
(6) In the homologous domain related function analysis, published original files of three databases of gene function: KEGG (http://www.genome.jp/kegg/) provided by Kyoto University, Pfam (http://pfam.sanger.ac.uk/) provided by Wellcome Trust Sanger Institute, and GO (http://www.geneontology.org) provided by Gene Ontology Consortium were integrated and used in the system for use.
(7) In order to visualize a Scalable Vector Graphics (SVG) language file (W3C 2003), known graphics software which interprets the SVG language or an Internet browser plug-in compatible with XML/SVG language form is used.
(8) The public software: Graphviz (http://www.graphviz.org/) was used as a network automatic layout tool in the program.

Hereinafter, various analysis results obtained by using the software for analyzing genome structure will be described again in detail with applied examples.

### <First modified example>

A first modified example of the information processing apparatus and the information processing method according to the present embodiment will be described briefly.

The information processing apparatus and the information processing method according to the present embodiment involves, for example, the steps of focusing on strings such as gene sequences, aligning the strings, extracting all similar sections (namely, homologous domains) of strings, and classifying the extracted homologous domains into families from the similarity relationship and geographical structure of the extracted homologous domains.

As for the families of the homologous domains obtained by the information processing apparatus and the information processing method according to the present embodiment, identification information such as an identification number (ID) can be applied to each of the extracted families. Then, the homologous domain phylogeny analyzing unit 103 according to the present modified example produces a string representing the occurrence order of the families using the IDs assigned to the extracted families and further applies the method of analyzing homologous domain phylogeny to the string of the occurrence order. This allows for analysis of higher-level families. Further the number of levels of the application of the method of analyzing homologous domain phylogeny is not particularly limited and it may be suitably set depending on a target string and analysis conditions.

In this case, the homologous domain phylogeny analyzing unit 103 can suitably use the known common distance metrics previously illustrated without limiting the distance metric to be used for performing sequence alignment on strings (number sequence) of identification numbers to a special distance metric such as a distance metric specialized to alignment of gene sequences.

Thus, when the method of analyzing homologous domain phylogeny is applied hierarchically, the relationship and structure of the homologous domain families which are separated by the insertion of many elements in the process of evolution and are not significant in a first hierarchy (i.e., a first application of the method of analyzing homologous domain phylogeny to gene sequences) can be more specifically revealed. As a result, the original form of the string structure, which was originally present, can be searched, so that the transition of the chromosome rearrangement can be more specifically revealed.

The hierarchical application of the homologous domain analysis method described above will be specifically described with applied examples.

### (Hardware configuration)

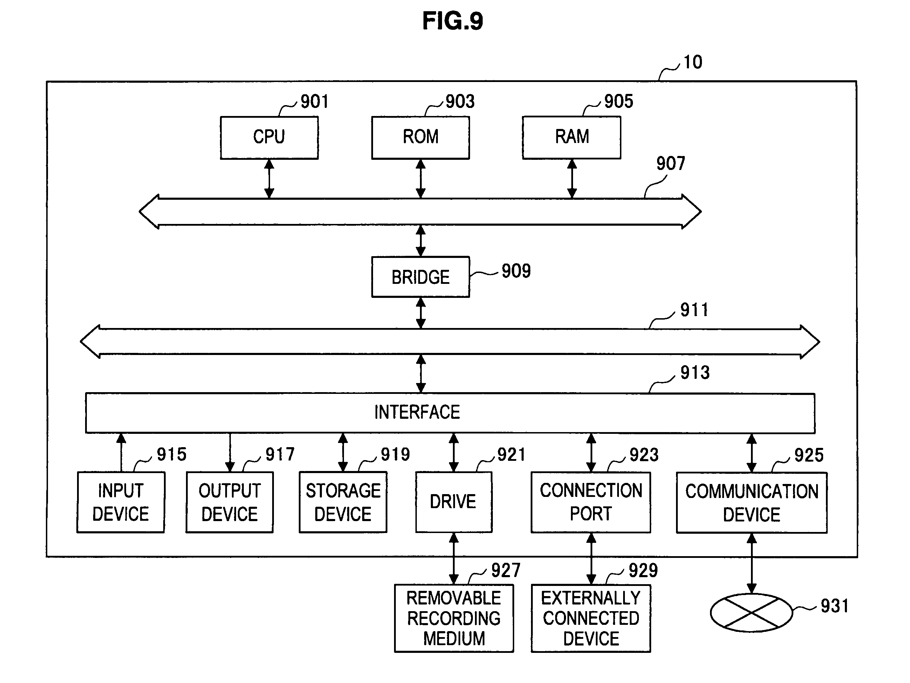

Subsequently, the hardware configuration of the information processing apparatus 10 according to the embodiments of the present invention will be described in detail with reference to FIG. 9. FIG. 9 is a block diagram for explaining the hardware configuration of the information processing apparatus 10 according to the embodiments of the present invention.

The information processing apparatus 10 mainly includes a CPU 901, a ROM 903, and a RAM 905. Further, the information processing apparatus 10 includes a host bus 907, a bridge 909, an external bus 911, an interface 913, an input device 915, an output device 917, a storage device 919, a drive 921, a connection port 923, and a communication device 925.

The CPU 901 functions as a processing and a control unit, and it controls the whole or a part of operation in the information processing apparatus 10 according to various programs stored in the ROM 903, the RAM 905, the storage device 919 or a removable recording medium 927. The ROM 903 stores a program to be used by the CPU 901, a processing parameter and the like. The RAM 905 primarily stores programs used by the CPU 901 in the execution, parameters and the like that are appropriately changed during the execution. The CPU 901, the ROM 903 and the RAM 905 are connected with one another through the host bus 907, which is an internal bus such as a CPU bus.

The host bus 907 is connected to the external bus 911 such as a Peripheral Component Interconnect/Interface (PCI) bus via the bridge 909.

The input device 915 is an operating mechanism to be operated by a user, such as a mouse, a keyboard, a touch panel, buttons, a switch or a lever, for example. For example, the input device 915 may be a remote controlling mechanism (so-called remote control) with an infrared ray or another radio wave, or an externally connected device 929 compatible with the operation of the information processing apparatus 10, such as a cellular phone or a PDA. Further, the input device 915 includes an input control circuit that generates an input signal based on information input by a user using the above operating mechanism and outputs it to the CPU 901, for example. By operating the input device 915, a user of the information processing apparatus 10 can input various data or give an instruction of a processing operation to the information processing apparatus 10.

The output device 917 includes an apparatus capable of visually or audibly notifying obtained information to the user. Examples of such apparatus include a display device such as a CRT display device, a liquid crystal display device, a plasma display device, an EL display device or a lamp, an audio output device such as a speaker or a headphone, or a printer, a cellular phone or a facsimile. The output device 917 outputs, for example, results obtained by various processing by the information processing apparatus 10. Specifically, the display device displays a result obtained by various processing of the information processing apparatus 10 as a text or an image. The audio output device converts an audio signal containing reproduced audio data, acoustic data or the like into an analog signal and outputs it.

The storage device 919 is a device for data storage that is configured as an example of a storage unit of the information processing apparatus 10. The storage device 919 includes a magnetic storage device such as a hard disk drive (HDD), a semiconductor storage device, an optical storage device, a magneto-optical storage device or the like. This storage device 919 stores a program to be executed by the CPU 901, various data, or various data acquired from the outside.

The drive 921 is a reader/writer for a recording medium, which is built in the information processing apparatus 10 or attached thereto. The drive 921 reads information that is recorded in the removable recording medium 927 such as a magnetic disk, an optical disk, a magneto-optical disk or semiconductor memory which is attached thereto and outputs the information to the RAM 905. Further, the drive 921 can write information into the removable recording medium 927 such as a magnetic disk, an optical disk, a magneto-optical disk or semiconductor memory which is attached thereto. Examples of the removable recording medium 927 include a DVD medium, an HD-DVD medium, and a Blu-ray medium. In addition, examples of the removable recording medium 927 include a compact flash (registered trademark) (CF), a flash memory, and a secure digital (SD) memory card. Further, the removable recording medium 927 may be an integrated circuit (IC) card equipped with a contactless IC chip or an electronic appliance.

The connection port 923 is a port for directly connecting devices to the information processing apparatus 10. Examples of the connection port 923 include a universal serial bus (USB) port, an IEEE 1394 port, and a small computer system interface (SCSI) port. In addition, examples of the connection port 923 include an RS-232C port, an optical audio terminal, and a high-definition multimedia interface (HDMI) port. By connecting the externally connected device 929 to the connection port 923, the information processing apparatus 10 can directly acquire various data from the externally connected device 929 or supply various data to the externally connected device 929.

The communication device 925 is a communication interface that is constituted by a communication device or the like for connecting to a communication network 931, for example. The communication device 925 may be a communication card for wired or wireless local area network (LAN), Bluetooth (registered trademark), or wireless USB (WUSB). Alternatively, the communication device 925 may be a router for optical communication, a router for asymmetric digital subscriber line (ADSL), or a modem for each kind of communication. The communication device 925 can transmit and receive a signal or the like in conformity to a prescribed protocol such as TCP/IP on the Internet or with other communication devices, for example. Further, the communication network 931 that is connected to the communication device 925 includes a wired or wireless network or the like, and it may be the Internet, home LAN, infrared data communication, radio wave communication, satellite communication or the like.

An example of the hardware configuration that can realize the functions of the information processing apparatus 10 according to each embodiment of the present invention has been described in the foregoing. Each of the constituent elements may be constituted using a general-purpose part, or it may be constituted by hardware specialized to the function of each constituent element. It is thereby possible to change the configuration to be used as appropriate according to the technique level available when implementing the embodiment.

### (Applied examples)

The software for analyzing genome structure using the method of analyzing homologous domain phylogeny was used to perform various analyses. Hereinafter, the performed analyses and the results will be described.

### <Applied example 1: analysis of genome structure of Cyanobacteria PCC6803>

The genome (all main chromosomes and endogenous plasmids) of Cyanobacteria: Synechocystis sp. PCC6803 (Kaneko et al., DNA Research, 3:109-136(1996), Kaneko et al., DNA Research, 10:221-228(2003)) were analyzed using the software for analyzing genome structure.

### Analytical object

As genome sequence information, completely, sequences (the 1-st to 7-th sequences) among eight types of DNAs shown in TABLE1, which had been discovered from PCC6803 strain and published were used in the analysis. Sequence alignment of 7 kinds of Self-Self combinations and 21 kinds of Self-Other combinations was performed on these seven sequences using the published alignment software: BLAST, and the output results that were combined to be a text data of about 2.6 MB were used as an object for the homologous domain phylogeny analysis. A region margin ratio of 3% (MARGIN = 3) was given as an argument.

[Table 1]

### Results

The analysis output results are summarized statistically in TABLE 2 below. 2354 homologous sections (locus) are extracted and these sections are regionally hierarchized and present in 352 independent zones: the large region (domain). It is found that each of the large regions belongs to one of the 76 large families (BDF). As for pCA2.4 and pCB2.4, no homologous section was extracted in any of the Self-Self and Self-Other combinations.

[Table 2]

In the software for analyzing genome structure according to the present embodiment, it is possible to produce a physical map of extracted genes (encoding regions) and homologous domains. In the physical map, each DNA is shown as a double strand and the genes (or encoding regions) on respective chains can be indicated in black. The homologous domain can be shown as three level hierarchy boxes sticking out from one of the chains. Among the three level hierarchy boxes, the outermost box shows a large region (domain), the box inner shows a middle region (ceiling), and the innermost box located at the outside of the gene (encoding region) shows a small region (region). Phylogenic families discovered by the software for analyzing genome structure can be illustrated in different colors.

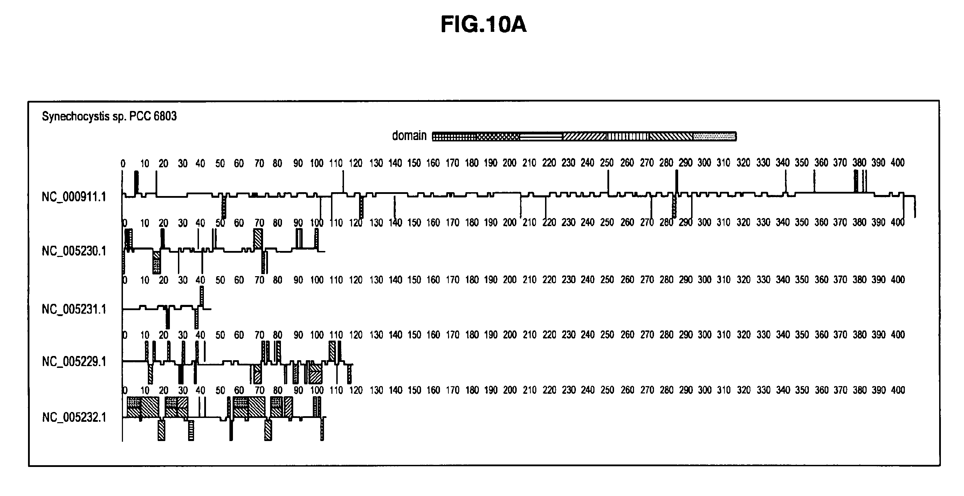

FIG. 10A schematically illustrates a part of the physical map of the genes and homologous domains illustrated above. Here, an interesting discovery easily observed from the display according to the patterns of the families will be described. NC_005232.1 displayed on the lowest stage shows an analysis result that there are two blocks of similar patterns connected to each other where the family of a homologous large region (the outermost hierarchy) is similar (the order in the range from 0 to around 30 and the order in the range from 60 to around 90). This shows a trace that plasmid pSYSX was originally a half in size and the whole plasmid was replicated at a certain time. Further, homologous domains of smaller sizes and of the same families as seen in NC_005232.1 belonging to the same family are observed in NC_005229.1 of the next upper stage. This suggests that homologous recombination has occurred between the two plasmids.

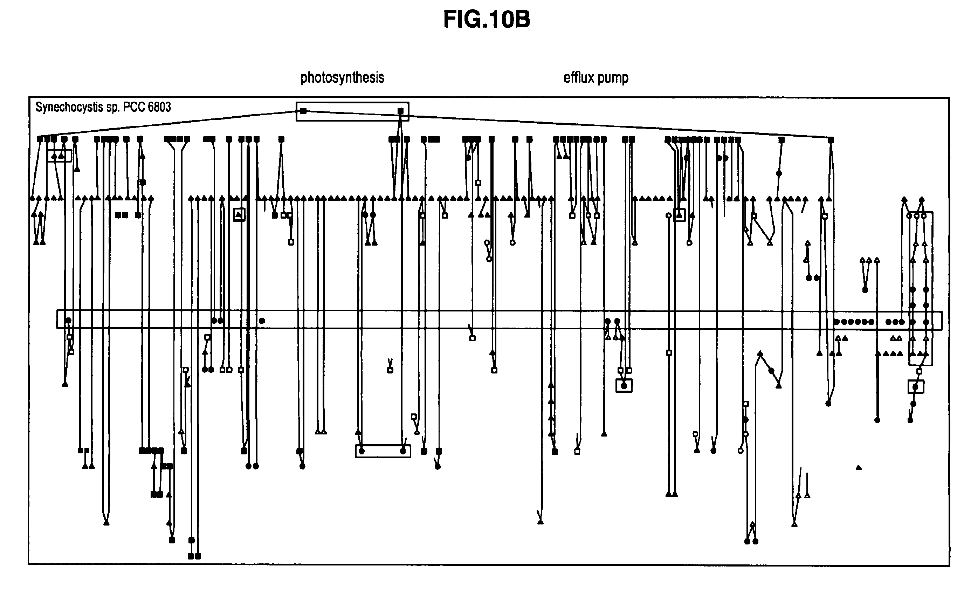

It is also possible to output a scheme diagram in which each of the homologous domains is automatically laid out based on the occurrence order and family information without depending on the size. In FIG. 10B, a scheme diagram output from the software for analyzing genome structure according to the present embodiment is illustrated. In FIG. 10B, each DNA molecule is displayed starting from the left, in the same order as shown in the physical map. When NC_005232.1 at the right end of the drawing is observed, repetitive patterns of the family are more clearly seen. In NC_005229.1 on its left side, many homologous domains of the same families and many patterns thereof are found. The possibility that the two plasmids have been mutually recombined is apparent from the drawing.

Although not illustrated in the present specification, it is also possible to output a phylogenetic tree formed by subdividing the phylogeny of a BD family by the hierarchical family structure of the large, middle, and small regions. Each family can be represented as a radial phylogenetic tree where the BD family, BDC family, BDCR family, and large region (domain) belonging to these families are drawn in order from the center to the outside in a radial fashion.

Although not illustrated in the present specification, it is also possible to output an internal configuration diagram of the homologous domains belonging to each of the BD families in individual DNA molecules. It is found that the phylogeny of the BD family can be subdivided from the internal configuration of the homologous domains belonging to the same family by referring to the internal configuration diagram. For example, the 40th BD family (F40) includes 93 (the largest number of) homologous domains. Among them, 81 homologous domains were found on chromosome (NC_000911.1), 3 homologous domains were found on pSYSM (NC_005229.1), 4 homologous domains were found on pSYSA (NC_005230.1), 4 homologous domains were found on pSYSG (NC_005231.1), and 1 homologous domain was found on pSYSX (NC_005232.1).

The function analysis results regarding the 76 BD families (large family) are shown in TABLE3. It is found that 8 families include genes homologous to transposase and are derived from transposon. A short sequence peculiar to transposon is referred to as an insertion sequence (IS). The insertion sequences so far discovered in various bacteria have been classified. Three insertion sequences, IS4, IS5, and IS630-Tcl-mariner have been experimentally discovered from PCC6803 (Cassier-Chauvat et al., Gene, 195:257-266 (1997)). As a result of the analysis, it is found that these insertion sequences are included in the homologous large region of the 40th BD family. It is found that 3 families are involved in cell division and replication, 7 families are involved in photosynthesis and carbon fixation, 2 families are involved in transcription, 4 families are involved in translation, 2 phylogenies are involved in methylation, 2 phylogenies are involved in phosphorylation, 6 families are involved in outer membrane system, and 5 phylogenies are involved in other enzymes.

[Table 3-1]

[Table 3-2]

### <Applied example 2: use for genetic engineering>

Under the assumption of replacing genes present in (chromosome, NC_000911.1) of the cyanobacteria PCC6803 with a foreign gene, the presence of the homologous domain located around sll0721 gene being focused was examined using the software for analyzing genome structure described previously.

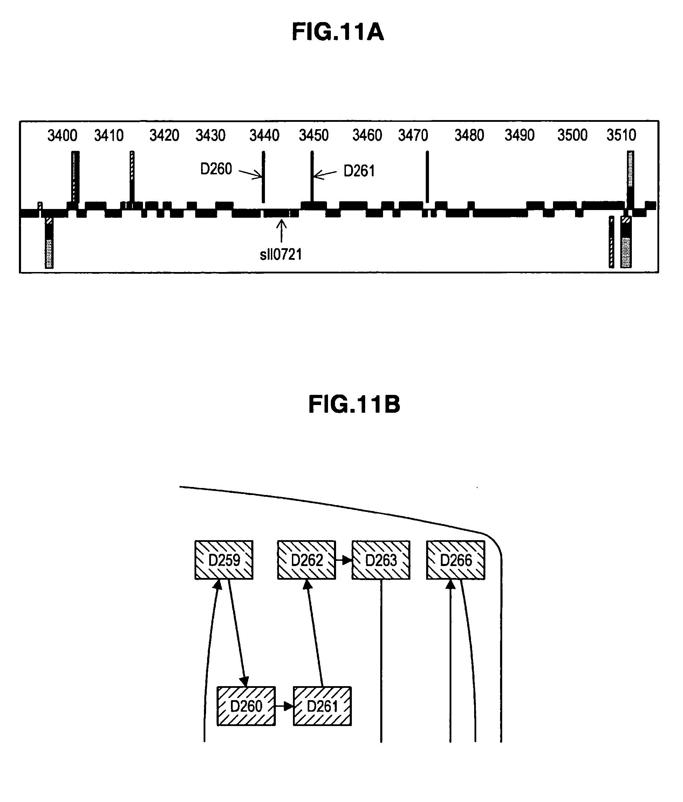

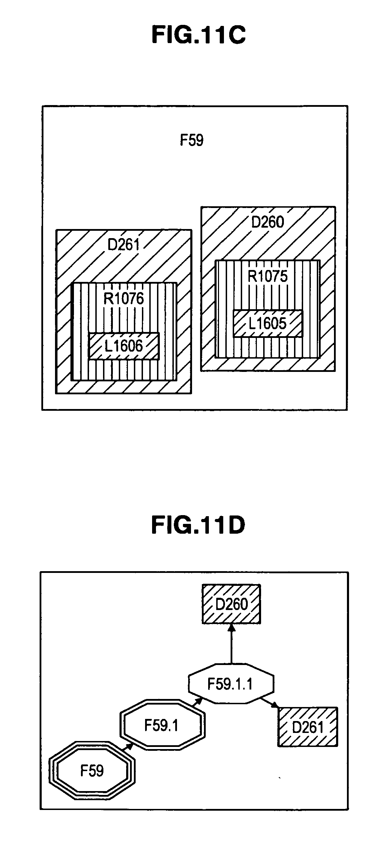

A physical map and a sequence diagram of homologous domains around gene sll0721 (coded region address: 3443514-3447386) are enlarged and shown in FIGS. 11A and 11B, respectively. A homologous domain D260 was detected 1 kb upstream from the 5'end of sll0721 and a homologous domain D261 was detected 3.3 Kb downstream from the 3'end of sll0721. As illustrated by an internal configuration diagram of the homologous domains shown in FIG. 11C and a family phylogenetic tree of the homologous domains shown in FIG. 11D, it is found that the homologous domain D260 of 122 bp in length and the homologous domain D261 of 123 bp in length belong to the 59th family and there is no other homologous domains belonging to the family.

From the homologous domain function analysis, it is found that the homologous domain D261 is present inside the s1r1651 (heamolysin secretion ATP-binding protein) gene (Sakiyama et al., J. Bacteriol., 188:3535-3542 (2006)), the region between the homologous domains D260 and D261 forms an operon composed of hemolysin secretion-related genes, including sl10721 (leukotoxin: LtA), an operon composed of circadian clock related genes is placed upstream of the homologous domain D260, and an amino acid amidohydrolase gene is placed downstream of the homologous domain D261.

The upstream and the downstream are considered not to be involved in hemolysin. Thus, it is estimated that the region between the homologous domains D260 and D261 is a region integrated into the genome by homologous recombination in the process of evolution. Therefore, it is estimated that when the homologous domains D260 and D261 are left behind and a gene structure is inserted to replace a region between the domains or when a foreign gene is inserted between the homologous domains D260 and D261, the inserted gene structure and foreign gene may be lost by homologous recombination in the future.

Thus, it is clear that the genome structure analysis based on the method of analyzing homologous domain phylogeny according to the present embodiment provides the detailed information associated with not only the whole genomic structure but also the local genomic structure including the unstable region and supports genetic engineering.

### <Applied example 3: use for biological evolution analysis>

Organisms with the photosynthetic function synthesize a pigment constituting an optical absorber in the cell. Focusing on this point, a phylogenetic tree of the photosynthetic organisms which are phylogenetically classified by the structure of the pigment held has been reported (Xiong et al. Science, 289:1724-1730 (2000)). According to the phylogenetic tree, archaea generating methane, purple non-oxygenic photosynthetic bacteria including Rhodobacter sphaeroides with bacteriochlorophyll a, green photosynthetic bacteria including Chlorobium tepidum with bacteriochlorophyll c, halobacteria with bacteriochlorophyll g, cyanobacteria (oxygenic photosynthesis bacteria with chlorophyll a), and plants appeared on the earth in this order.

Further, a phylogenetic tree of cyanobacteria which is phylogenically classified by 16S rRNA is reported (Tomitani et al., PNAS, 13 (14):5442-5447 (2006)). According to the phylogenetic tree, Gleobacter species are considered to be the most ambient cyanobacteria, followed by Synechococcus elongatus species which are autotrophic bacteria (CO₂ in the atmosphere or HCO₃⁻ in water is used as the only source of carbon) and Synechocystis species which are heterotrophic bacteria (sugar such as glucose is absorbed as a source of carbon in addition to CO₂ in the atmosphere or HCO₃⁻ in water). Appearance of Anabaena and Nostoc species with the nitrogen fixation ability in addition to the carbon fixation ability is considered to be much later in the evolution.

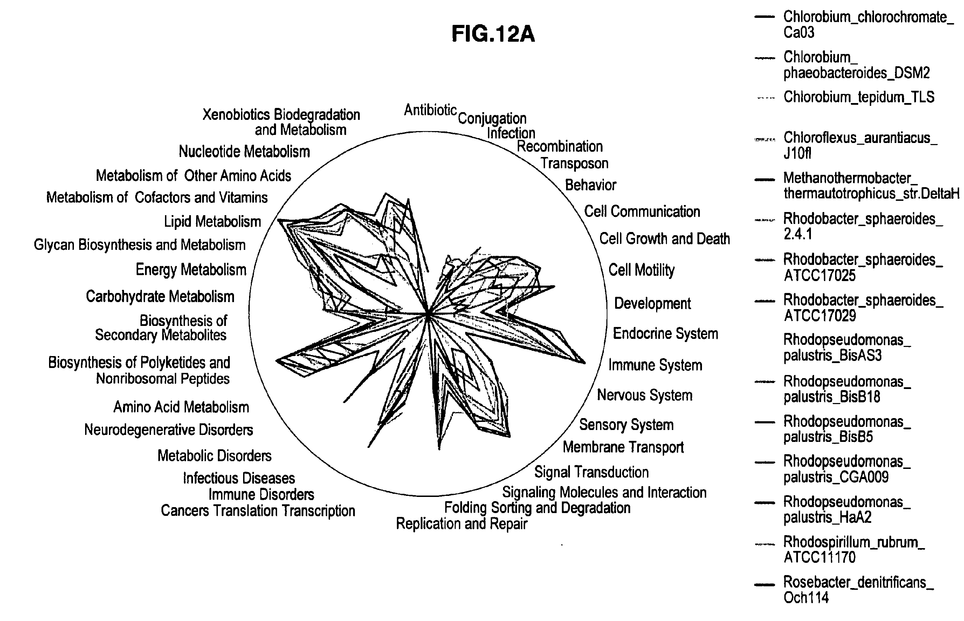

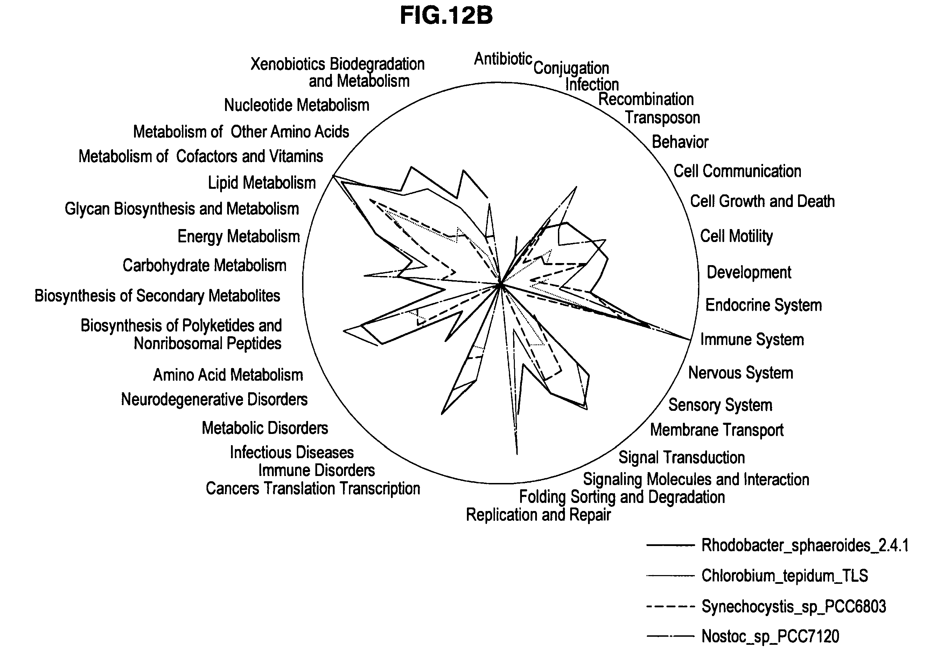

The genome structure analysis based on the method of analyzing homologous domain phylogeny according to the present embodiment was performed on 15 strains of non-oxygenic photosynthetic bacteria. FIGS. 12A and 12B are star-shaped graphs showing the number of homologous domain families classified by the function, obtained as the analysis result.

Colors of purple photosynthetic bacteria including Rhodobacter sphaeroides, red photosynthetic bacteria including Rosebacter denitrifiacans, green photosynthetic bacteria including Chlorobium tedium were arranged depending on the pigments thereof (where they are presented in black and white in FIG. 12A.). As is clear from FIG. 12A, curves showing purple photosynthetic bacteria appeared in the outermost area of the star-shaped graph. Inside the curve, curves showing green photosynthetic bacteria appeared. In the innermost area, curves showing red photosynthetic bacteria appeared. In several functional classes (Infection, Recombination, Transposon, Behavior, Cell Communication, Cell Growth and Death, and Cell Motility), intersection of the curves is observed. In the other functional classifications, purple line curves in the outermost and green line curves group are grouped to have mutually similar shapes. On the other hand, Rosebacter denitificans represented by a red curve has a shape different from those of the purple and green curves. In nearly all of the functional classes, the purple curves stay outside only in the functional classes of Transposon, Chlorobium phaeobacteriodes of Clorobium species shows a maximum value. This suggests the possibility that functional acquisition and genomic rearrangement may be frequently caused by introduction of foreign genes. In the example shown in FIG. 12B, as with the case shown in FIG. 12A, it is found that the shapes of the curves appeared in the star-shaped graph are grouped for each type of the pigments included in photosynthetic bacteria.

These examples show that information useful for elucidation of biological evolution can be obtained by the genome structure analysis based on the method of analyzing homologous domain phylogeny according to the present embodiment.

### <Applied example 4: use for resolution of pathology>

The genome structure analysis using the method of analyzing homologous domain phylogeny according to the present embodiment was performed on nonpathogenic Escherichia coli K12 strain used for study, and three types of pathogenic bacteria: Escherichia coli O157:H7 strain known as a virulent pathogenic strain, and Pseudomonas aeruginosa PA7 strain having resistance to a wide range of drugs, and Pseudomonas putida f1 having resistance to solvents such as toluene.

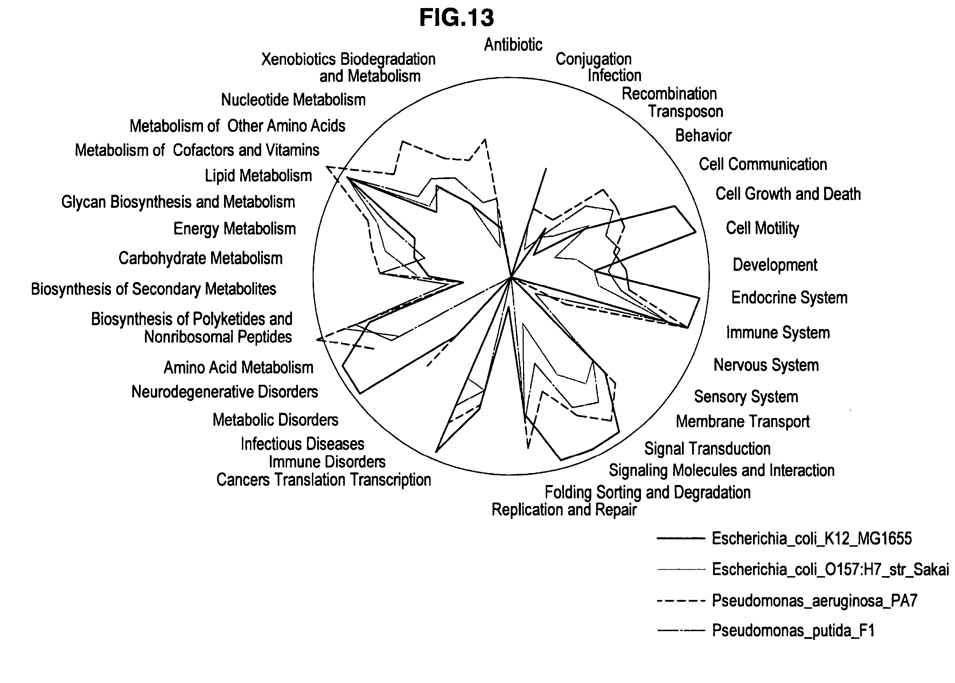

FIG. 13 shows a star-shaped curve where each point indicates the number of homologous domain families classified by the function, obtained as the analysis result. It is found that curves of three types of pathogenic bacteria: Escherichia coli O157: H7, P.aeruginosa PA7 strain, and P.putida f1 show a similar figure and are largely different from the curve (thick line) of Escherichia coli K12 strain for study. Significant differences are observed in the following functional classes: Cell Growth and Death, Cell Motility, Endocrine System, Signaling Moleculesand Interaction, Folding Splitting and Degradation, and Infectious Diseases.

These functional classes are information characterizing pathogenic bacteria. In the functional classes, the analysis results that enteropathogenic Escherichia coli was different from non-pathogenic Escherichia coli for study and showed behavior closer to that of the genus Pseudomonas were obtained. This shows that information useful for elucidation of pathology can be obtained by the genome structure analysis based on the method of analyzing homologous domain phylogeny according to the present embodiment.

### <Applied example 5: application to string structure analysis in other fields>

As described above, in the method of analyzing homologous domain phylogeny according to the present embodiment, a homological group of physically dispersed homologous domains and a regional group of geographically adjacent homologous domains are formed from homologous domains obtained by the sequence alignment of strings. Then, in the method of analyzing homologous domain phylogeny according to the present embodiment, the homological group and the regional group are combined to form a family and the family is subdivided from the regional configuration. Thus, the method of analyzing homologous domain phylogeny according to the present embodiment is a method based on a general concept. The string to be analyzed may be any string, such as a general document file, coded multimedia information, and time series data, in addition to genetic sequences.

For example, when the string information being focused is entity data of music contents, the method of analyzing homologous domain phylogeny according to the present embodiment can be used to analyze a chord progression of chord sounds. When the string information being focused is moving image contents including image contents and voice contents, for example, the presence of VIDEO editing can be analyzed by using the method of analyzing homologous domain phylogeny according to the present embodiment. In addition to these examples, the method of analyzing homologous domain phylogeny according to the present embodiment can be applied to the information with a certain kind of series.

### <Applied example 6: multi-hierarchy of method of analyzing homologous domain phylogeny>

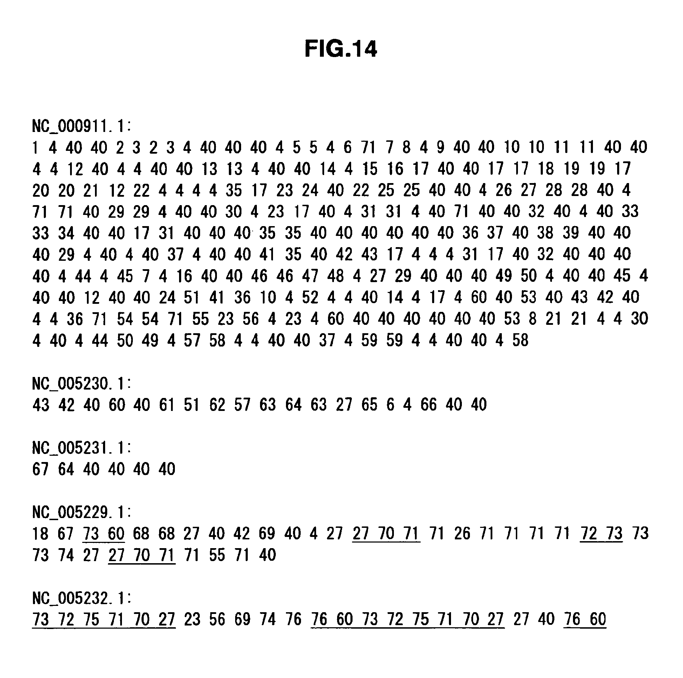

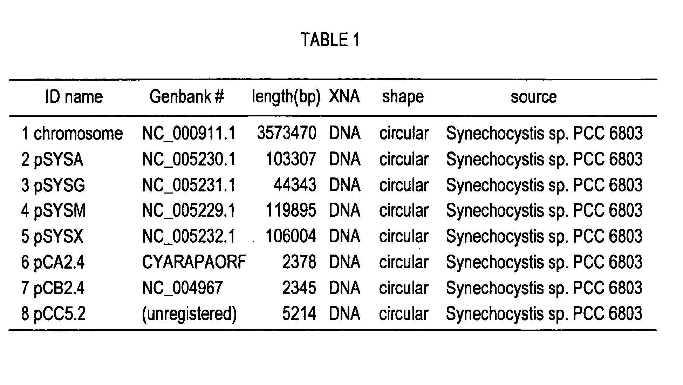

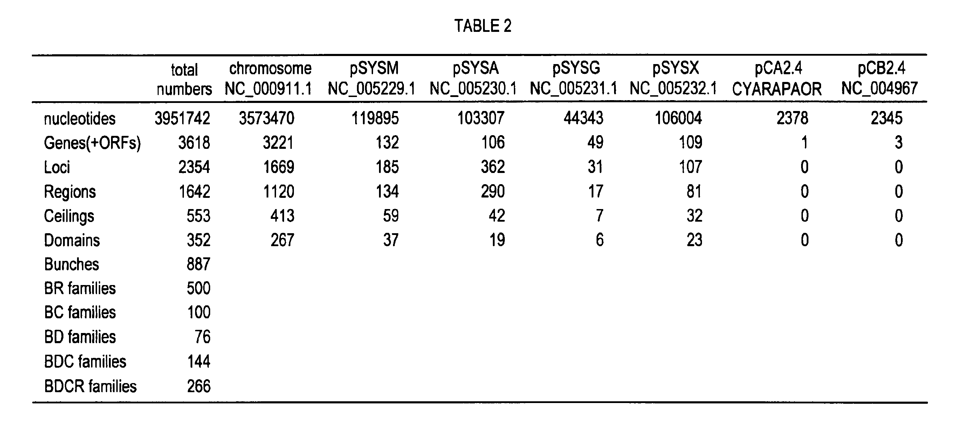

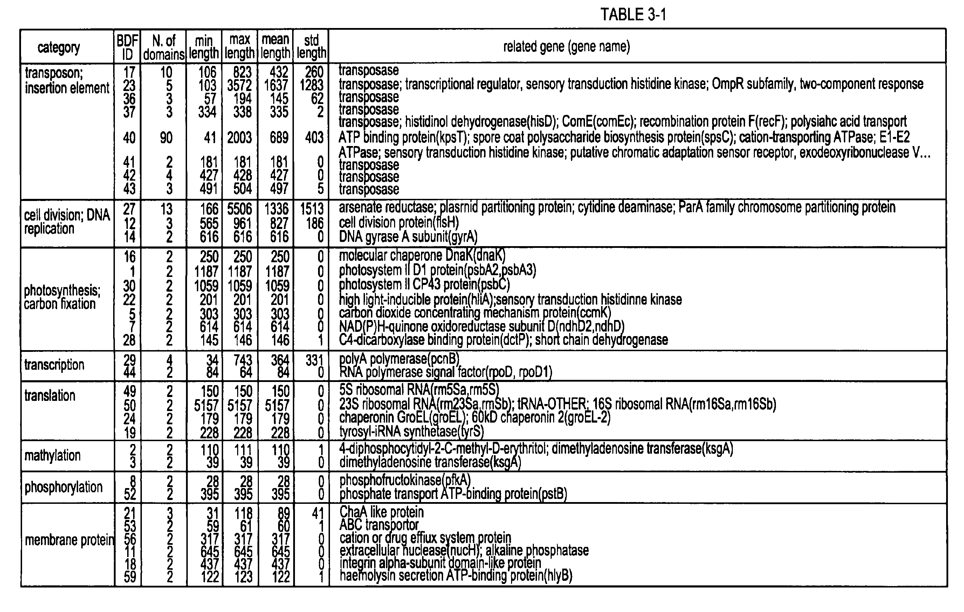

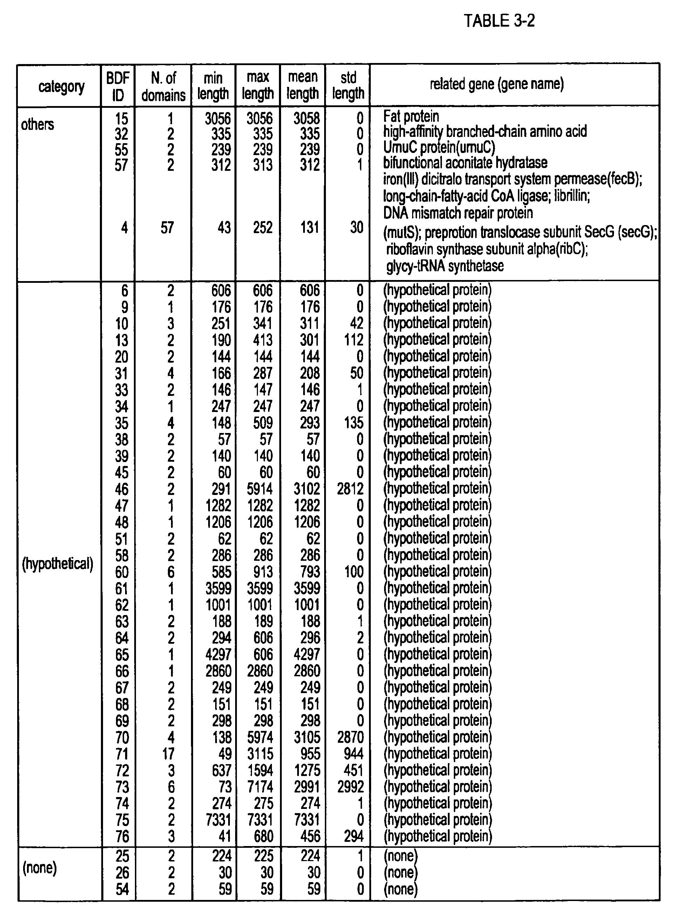

Subsequently, the results obtained by performing the analysis of higher-level families by applying the method of analyzing homologous domain phylogeny to multi-level hierarchies will be specifically described with reference to FIG. 14. In the Applied example, the families were further analyzed using the analysis result of the genome structure of cyanobacteria PCC6803 as an analytical object in Applied example 1.

As indicated in the Applied example 1, regarding Cyanobacteria PCC6803, 76 BD families (large family) were extracted by using the method of analyzing homologous domain phylogeny according to the embodiments of the present invention. Here, the physical map that is the original of the schematic diagram shown in FIG. 10A is a map visualized in the way that the homologous domains classified into 76 families are shown as up-and-down pulses and the domains are present having a size at a certain physical position on the genomes. The scheme diagram that is the original of the schematic diagram shown in FIG. 10B is a map in which the homologous domains are arranged with the family information in the occurrence order excluding the information of physical position and size.

In the Applied example, in the scheme diagram that is the original of the schematic diagram shown in FIG. 10B, the occurrence order of the homologous domain families (BD families) was recognized as a set of five types of strings (so to speak, the second hierarchy's string) composed of the family IDs assigned to each of the families (in the Applied example, an integer of 1 to 76) as elements and was handled as a set of string data as shown in FIG. 14. Namely, in the Applied example, the data of the five types of strings were further analyzed by the software for analyzing genome structure according to the present embodiment, the ID sequences of homologous domain families were extracted as a higher-level homologous domain, and the extracted higher-level families were analyzed.

In Applied example 1 above, it has been described that a certain pattern of homologous domain families appears twice in the 5th molecule NC_005232.1 (the lowest stage in FIG. 10A, the molecule at the rightmost end in FIG. 10B). The site corresponds to the underlined pattern "76 60 73 72 75 71 70 27" (homologous domain family sequence) in the string shown on the lowest stage in FIG. 14. This pattern can be recognized as the second hierarchy's homologous domain. As indicated by underlining in FIG. 14, it is clear that a fragment of "76 60" at the end and a fragment of "73 72 75 71 70 27" at the beginning are bound on a circular molecule to form the pattern "76 60 73 72 75 71 70 27".

In Applied example 1, it has been described that the homologous domain family pattern extracted from the 5th molecule NC_005232.1 appears in the molecule NC_005229.1. It is confirmed that the site corresponds to underlined portions in the string data shown in the second string from the bottom in FIG. 14. Hence, the pattern "76 60 73 72 75 71 70 27" can be identified as fragments of family sequences such as a pattern "27 70 71", a pattern "73 60", and a pattern "72 73" in a string pattern of molecule NC_005229.1 shown in FIG. 14. The result shows that, in the homologous domain family ID sequence of molecule NC_005229.1, the pattern "27 70 71", the pattern "73 60", and the pattern "72 73" belong to the same higher-level family as those of the pattern "76 60 73 72 75 71 70 27" in molecule NC_005232.1.

### (Summary)

As described above, in the embodiments of the present invention, the method of analyzing homologous domain phylogeny which is a general procedure to analyze the distribution, structure, and phylogeny of similar string pieces (homologous domains) in a set of one or more strings is proposed, and the basic concept and the analysis procedure are clarified. Further, the software for analyzing genome structure realized by using the analysis method, the genome structure analysis of cyanobacteria strain using the software for analyzing genome structure, and various applicable fields have been described.

In the method of analyzing homologous domain phylogeny described above, without focusing on any specific gene, in other words, without introducing elements with biological interpretation, all homologous domains are extracted from strings of genetic sequences by the mechanical operation of self-alignment. Thereafter, families of the homologous domains are formed from the regional structure and homological relationship without knowing (unknown) what the extracted homologous domains are till the end. After the families are formed, it is possible to analyze positional relationship of the homologous domains in genes and determine the family function by checking the individual families against the gene information. Thus, the analysis method is not the genome alignment between different organisms, but a method capable of searching for the evolution from the self-alignment of one genome.

The analysis method and the software reveal the possibility of the transition and rearrangement of the genome in the biological field, support genetic engineering, and provide information useful for eludiation of pathology and biological evolution. The analysis method is general enough and is a highly valuable procedure which can be used for not only the analysis of the structure of genetic sequences but also the analysis of the structure of strings in other fields.

It should be understood by those skilled in the art that various modifications, combinations, sub-combinations and alterations may occur depending on design requirements and other factors insofar as they are within the scope of the appended claims or the equivalents thereof.
The present application contains subject matter related to that disclosed in Japanese Priority Patent Application JP 2010-119915 filed in the Japan Patent Office on May 25, 2010 and Japanese Priority Patent Application JP 2011-026371 filed in the Japan Patent Office on February 9, 2011, the entire content of which is hereby incorporated by reference.

## Claims

1. An information processing apparatus comprising:
a data acquiring unit which acquires string data representing a string of one or more characters; and
a phylogeny analyzing unit which analyzes the string data acquired by the data acquiring unit to extract homologous string pieces in a string represented by the string data and performs phylogeny analysis based on the regional relationship and homological relationship of the extracted homologous string pieces.

2. The information processing apparatus according to claim 1,
wherein the phylogeny analyzing unit further includes
a sequence aligning unit which performs sequence alignment on a plurality of string data and calculates the similarity measure between the string data,
a homologous domain extracting unit which extracts homologous domains including homologous section information representing sections of the homologous string pieces and homological relationship information represented by using at least one of the direction and homogeny degree of the sections of the homologous string pieces using the sequence alignment result by the sequence aligning unit,
a homological group analyzing unit which analyzes the homologous domains dispersedly present in the same string data or between the different string data and groups homologous domains having common homologous section information among the homologous domains into a homological group, a regional group analyzing unit which analyzes the homologous domains dispersedly present in the same string data or between the different string data and produces a regional group representing inclusion and overlap relationships between the homologous domains, and
a family analyzing unit which analyzes a family of the homologous string pieces based on information about the homological group grouped by the homological group analyzing unit and information about the regional group produced by the regional group analyzing unit.

3. The information processing apparatus according to claim 2,
wherein the regional group analyzing unit sets a region obtained by adding a predetermined length of margin section to both ends of the sections of the homologous string pieces as a small region, sets the small region which is included in neither of the other small regions as a middle region, and sets a set of the middle region overlapped as a large region.

4. The information processing apparatus according to claim 3,
wherein the family analyzing unit analyzes the small region, the middle region, and the large region produced by the regional group analyzing unit and estimates a small region family representing a family of the small region, a middle region family representing a family of the middle region, and a large region family representing a family of the large region.

5. The information processing apparatus according to claims 1 to 4,
wherein the phylogeny analyzing unit further includes a family subdividing unit which subdivides the large region family.

6. The information processing apparatus according to claims 1 to 5,
wherein the phylogeny analyzing unit further includes a direction determining unit which determines the alignment direction of a section of the homologous string piece which belongs to the same homological group and the same family among the sections of the homologous string pieces belonging to the small region, the middle region, and the large region.

7. The information processing apparatus according to claims 1 to 6, further including a function analyzing unit which analyzes a function of the homologous domain based on the analysis result by the phylogeny analyzing unit.

8. The information processing apparatus according to claims 1 to 7,
wherein the phylogeny analyzing unit further performs the phylogeny analysis on the string data representing the phylogeny analysis result of the extracted homologous string pieces.

9. An information processing method comprising the steps of:
acquiring string data representing a string of one or more characters;
analyzing the acquired string data to extract homologous string pieces in a string represented by the string data; and
performing phylogeny analysis based on the regional relationship and homological relationship of the extracted homologous string pieces.

10. A program for causing a computer to achieve:
a data acquisition function for acquiring string data representing a string of one or more characters; and
a phylogeny analysis function for analyzing the string data acquired by the data acquisition function, extracting homologous string pieces in a string represented by the string data, and performing phylogeny analysis based on the regional relationship and homological relationship of the extracted homologous string pieces.
